# EUROPEAN PATENT SPECIFICATION

(11) **EP 1 297 338 B1**
(45) Date of publication and mention of the grant of the patent: **18.04.2007**
(21) Application number: 01946078.1
(22) Date of filing: 04.06.2001
(51) Int. Cl.: G01N 33/543

(54) **MICROARRAYS FOR PERFORMING PROTEOMIC ANALYSES**
MIKROARRAYS FÜR DURCHFÜHREN VON PROTEOMANALYSE
MICRORESEAUX PERMETTANT DE REALISER DES ANALYSES PROTEOMIQUES

(30) Priority: 05.06.2000 US 209711 P
(43) Date of publication of application: 02.04.2003
(73) Proprietor: Novartis Vaccines and Diagnostics, Inc., Emeryville, CA 94608 (US)
(72) Inventor: CHARYCH, Deborah, Albany, CA 94706 (US); BEAUSOLEIL, Eric, San Francisco, CA 94131 (US); ZUCKERMANN, Ronald, N., El Cerrito, CA 94530 (US)
(74) Representative: UEXKÜLL & STOLBERG
(86) International application number: PCT/US2001/018066
(87) International publication number: WO 2001/094946

(56) References cited:
- WO-A-01/01142
- WO-A-92/10757
- WO-A-98/31839
- WO-A-98/59360
- WO-A-99/48897
- DE-A- 19 741 716
- US-A- 5 965 695
- MACBEATH G ET AL: "PRINTING SMALL MOLECULES AS MICROARRAYS AND DETECTING PROTEIN-LIGAND INTERACTIONS EN MASSE" JOURNAL OF THE AMERICAN CHEMICAL SOCIETY, AMERICAN CHEMICAL SOCIETY, WASHINGTON, DC, US, vol. 121, no. 34, 1 September 1999 (1999-09-01), pages 7967-7968, XP001056469 ISSN: 0002-7863
- PIRRUNG M C ET AL: "A General Method for the Spatially Defined Immobilization of Biomolecules on Glass Surfaces Using Caged Biotin" BIOCONJUGATE CHEMISTRY, AMERICAN CHEMICAL SOCIETY, WASHINGTON, US, vol. 7, no. 3, May 1996 (1996-05), pages 317-321, XP002095758 ISSN: 1043-1802

## Description

### BACKGROUND OF THE INVENTION

The present invention relates to cell product analysis and materials. In one embodiment, the invention is directed to proteomic microarrays and methods of using them to conduct proteomic analyses.

In recent years, microarray technology has developed from a specialized subfield into an important tool for basic and applied studies in molecular biology, microbiology, pharmaceutics, agriculture, and many other biotechnologies. DNA microarray technology attempts to link the genome of an organism or cell to an expressed phenotype or protein function.

The overwhelming publication and patent literature on micro array technology describes arrays of DNA (or other forms of nucleic acid, such as cDNA or RNA), displayed on a solid surface such as a glass slide (often referred to as a "chip"). The arrayed DNA is typically in the form of short oligonucleotides (e.g., about 8 to 25 bases) or longer clones or PCR products (about 500 to 2000 bases). The former are typically synthesized on the solid support, whereas the latter are robotically "spotted" onto a solid support into an array format.

While there are reports of peptide and protein arrays on solid surfaces, these have received considerably less attention in comparison to DNA arrays. This is likely due to the inherent instability of these materials at interfaces, and in the presence of complex biological matrices. For example, it is well known, that many proteins denature upon contact with solid surfaces. Peptides, as well as proteins, are also subject to hydrolysis by any proteases that may be present in the biological sample being analyzed. In addition, peptide arrays are typically synthesized *in-situ* on solid surfaces using photolithographic methods. These techniques require the use of expensive custom-made masks that must be designed and manufactured for each chip. Furthermore, chemical characterization of surface-synthesized peptides is nearly impossible to perform due to the tiny amount of peptide generated.

WO 98/31839 describes general methods for immobilizing biological substances on a solid support, wherein the substrates may be comprised of gold or aluminum.

WO 98/59360 discloses substrates for the preparation of arrays of biological molecules which may be covered with gold or silicon oxide and optionally derivatized with other bifunctional molecules such as carbodiimide or N-hydroxysuccinimide.

WO 92/10757 discloses the coupling of molecules to a solid substrate by use of an thiol-avidin-biotin-avidin linkage.

MacBeath et al., J. Am. Chem. Soc. (1999), Vol. 121, 7967-7968, describes the functionalization of glass slides with aminopropyl triethoxysilane and maleimide. The generation of arrays of molecules on beads distributed in individual wells and the covalent attachment of the slide surface is disclosed.

US Patent No. 5,965,695 provides libraries of peptoids which are useful in the identification of receptor interactions.

Currently, the most common way of analyzing the proteome of biological samples employs two-dimensional ("2-D") gel electrophoresis. This method is problematic because the results are very sensitive to the experimental protocol (for example, development time of the gel as well as other parameters). Therefore, it is very difficult to get reproducible data from 2-D gels. Also, the sensitivity of the silver stain used in these gels is limited, and is less than that of the fluorescent labels used in microarray technologies.

Thus, there is an overwhelming need to develop effective microarray technology that is useful in a protein context. In many cases, functional pathways cannot be directly linked to a particular gene. Proteins often undergo a variety of post-translational modifications, interactions, or degradations that ultimately determine function. Even the seemingly simple evaluation of a protein's abundance cannot be directly correlated with the level of corresponding mRNA. The only solution is to evaluate the state of the cell, tissue or organism at the protein level. Therefore, a high throughput format that allows rapid display of protein differentials in complex mixtures such as cells, tissues, serum, etc., would provide a powerful counterpart and complement to DNA microarray technology.

### SUMMARY OF THE INVENTION

To achieve the foregoing, the present invention provides peptidomimetic protein-binding arrays, their manufacture, use, and application. The protein-binding array elements of the invention include a peptidomimetic segment, an anchor segment and a linker segment connecting the peptidomimetic and anchor segment. The invention contemplates peptidomimetic array element library synthesis, distribution, and spotting of array elements onto solid planar substrates, labeling of complex protein mixtures, and the analysis of differential protein binding to the array. The invention also enables the enrichment or purification, and subsequent sequencing or structural analysis of proteins that are identified as differential by the array screen. Kits including proteomic microarrays in accordance with the present invention are also provided.

In one aspect, the invention pertains to an array of protein-binding agents stably attached to the surface of a solid support. The array includes a solid substrate having a substantially planar aluminium surface coated with a silicon dioxide coating, wherein the silicon dioxide coating has a thickness of about 200 Å to about 900 Å and is capable of enhancing amplification of a fluorescent signal, and a plurality of different protein-binding agents bound to the substrate. Each of the protein-binding agents includes an anchoring segment stably bound to the substrate surface, a peptidomimetic protein-binding segment, and a linker segment connecting and separating the anchoring and peptidomimetic segments.

In another aspect, the invention pertains to a method of making an array comprising a plurality of different protein-binding agents stably associated with the surface of a solid support. The method involves preparing for bonding a solid substrate having a substantially planar aluminium surface coated with a silicon dioxide coating, wherein the silicon dioxide coating has a thickness of about 200 Å to about 900 Å and is capable of enhancing amplification of a fluorescent signal, and contacting a plurality of different protein-binding agents with the substrate under conditions sufficient for the protein-binding agents to become bound to the substrate surface. Each of the protein-binding agents includes an anchoring segment stably bound to the substrate surface, a peptidomimetic protein-binding segment, and a linker segment connecting and separating the anchoring and peptidomimetic segments.

In another aspect, the invention pertains to a method of making an array comprising a plurality of different protein-binding agents stably associated with the surface of a solid support. The method involves generating a library of protein-binding agents in which each of the agents includes an anchoring segment stably bound to the substrate surface, a peptidomimetic protein-binding segment, and a linker segment connecting and separating the anchoring and peptidomimetic segments. The protein-binding agents are distributed from the library into individual storage receptacles for each different protein-binding agent, a plurality of the different protein-binding agents are prepared for binding to a solid substrate, a solid substrate having a substantially planar aluminium surface coated with a silicon dioxide coating, wherein the silicon dioxide coating has a thickness of about 200 Å to about 900 Å and is capable of enhancing amplification of a fluorescent signal for binding with a plurality of the different protein-binding agents is prepared, and an array is prepared as described herein.

A further aspect of the present invention pertains to a method of performing a differential binding assay. The method involves labeling proteins in a protein-containing biological sample solution, contacting an aliquot of the labeled protein-containing biological sample solution with an array as described herein, and analyzing the array to determine differential binding of proteins in the sample to protein-binding agents of the array.

Another aspect of the present invention pertains to a kit for use in performing a differential binding assay as described herein. The kit includes an array having a solid substrate having a substantially planar aluminium surface coated with a silicon dioxide coating, wherein the silicon dioxide coating has a thickness of about 200 Å to about 900 Å and is capable of enhancing amplification of a fluorescent signal with a plurality of different protein-binding agents bound to the substrate. Each of the protein-binding agents includes an anchoring segment stably bound to the substrate surface, a peptidomimetic protein-binding segment, and a linker segment connecting and separating the anchoring and peptidomimetic segments.

A further aspect of the present invention pertains to a mixed array of protein-binding agents stably attached to the surface of a solid support. The array includes a solid substrate having a substantially planar aluminium surface coated with a silicon dioxide coating, wherein the silicon dioxide coating has a thickness of about 200 Å to about 900 Å and is capable of enhancing amplification of a fluorescent signal, and a plurality of different protein-binding agents bound to the substrate. Each of the protein-binding agents includes an anchoring segment stably bound to the substrate surface, a peptidomimetic protein-binding segment, and a linker segment connecting and separating the anchoring and peptidomimetic segments. The array further includes a plurality of different antibodies bound to the substrate.

These and other features and advantages of the present invention will be presented in more detail in the following specification of the invention and the accompanying figures which illustrate by way of example the principles of the invention.

### BRIEF DESCRIPTION OF THE DRAWINGS

Figs. 1A and 1B schematically depict the structure of a protein-binding agent array element and array portion, respectively, in accordance with one embodiment of the present invention.
Fig. 1C depicts the molecular structure a protein-binding agent linking segment composed of ethylene oxides in accordance with one embodiment of the present invention.
Figure 2 schematically depicts a process of making an array having a plurality of different protein-binding agents stably associated with the surface of a solid support in accordance with one embodiment of the present invention.
Figure 3 schematically depicts the molecular structures of six 12-mer peptoid array elements in accordance with one embodiment of the present invention.
Figures 4A and 4B schematically depict alternative modes of binding a protein-binding agent to a solid support in accordance with one embodiment of the present invention.
Figure 5 schematically depicts a process for conducting a differential protein binding assay in accordance with one embodiment of the present invention.
Figure 6 schematically depicts a various processes for identifying and characterizing proteins identified in accordance with embodiments of the present invention.
Fig. 7A depicts the chemical formula for a NHS-LC-LC-biotin molecule used in a solution to coat aluminum slides to be used as a substrate in accordance with one embodiment of the present invention.
Fig. 7B depicts a representation of an avidin-derviatized aluminum slide spotted with a biotinylated protein-binding agent in accordance with one embodiment of the present invention.
Fig. 8 depicts a graph of results of size exclusion chromatography conducted to separate labeled protein from the unreacted dye prior to application of the labeled protein sample to a microarray in accordance with embodiments of the present invention.
Figs. 9A-9C depict scans of microarray chips bearing a library of 1,000 peptoid-based protein-binding agents used to prove the concept of the present invention.
Fig. 10 depicts biotinylated hexameric peptides (A through E) and the corresponding signal intensity obtained when the biotinylated peptides were spotted onto avidin-treated slides, blocked with casein, and probed with Cy5-labelled antiglu antibodies.
Fig. 11 depicts the dependence of signal strength on the mode of surface attachment of a peptide for two modes of surface attachment in accordance with the present invention.

### DESCRIPTION OF SPECIFIC EMBODIMENTS OF THE INVENTION

The materials and associated techniques and apparatuses of the present invention will now be described with reference to several embodiments. Important properties and characteristics of the described embodiments are illustrated in the structures in the text and in the accompanying drawings. While the invention will be described in conjunction with these embodiments, it should be understood that the invention it is not intended to be limited to these embodiments. On the contrary, it is intended to cover alternatives, modifications, and equivalents as may be included within the spirit and scope of the invention. In the following description, numerous specific details are set forth in order to provide a thorough understanding of the present invention. The present invention may be practiced without some or all of these specific details. In other instances, well known process operations have not been described in detail in order not to unnecessarily obscure the present invention.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this invention belongs.

### Introduction

The present invention provides peptidomimetic protein-binding arrays, in addition to methods for their manufacture, use, and application. The protein-binding array elements of the invention include a peptidomimetic segment (an example of which is a peptoid), linked to a solid support using a stable anchor. In one embodiment, the invention provides peptidomimetic array element library synthesis, distribution, and spotting of array elements onto solid, planar substrates, labeling of complex protein mixtures, and the analysis of single protein binding or differential protein binding by contacting labeled pure protein or complex protein mixtures to the peptidomimetic array. Such analysis may lead to the identification of novel therapeutic targets involved in diseases such as cancer, HIV or diabetes. These novel targets can then be utilized in high throughput screening assays to find new drug candidates. The invention also enables the enrichment or purification, and subsequent sequencing or structural analysis, of proteins that are identified as differential by the array screen. The arrays can also be used to identify new synthetic ligands for proteins of interest (for protein purification or development of high throughput assays) or synthetic antigens for antibodies of interest. They may also be used to find new enzyme inhibitors or other high affinity ligands for drug targets, or as initial scaffolds for new therapeutics. Kits including proteomic microarrays in accordance with the present invention are also provided.

### 1. Protein-Binding Agent Arrays

Peptidomimetic arrays in accordance with the present invention are composed of a number of different array elements comprising protein-binding agents attached to the surface of a solid support. The different protein-binding agent array elements each include an anchoring segment attached to the substrate surface, a peptidomimetic protein-binding segment, and a linker segment connecting and separating the anchoring and peptidomimetic segments. A "peptidomimetic" as used herein refers to nonpeptide synthetic polymers or oligomers that detectably interact with proteins or receptors in a manner analogous to protein-protein or protein-peptide physical and/or chemical interactions under assay conditions. The anchoring segment is typically attached to the substrate surface such that the association between the anchoring group and the substrate surface, and thus the concentration and density of the protein-binding agent on the substrate surface, is maintained during the processing to which the microarray is subjected under its normal operating conditions, for example, as described herein. The number of different chemical species of protein-binding agent present on the surface of the array is at least 2, or at least 10, or at least 100, and can be much higher, generally being at least about 1,000, or at least about 5,000 to about 50,000, for example, between about 5,000 and about 10,000, as described further below.

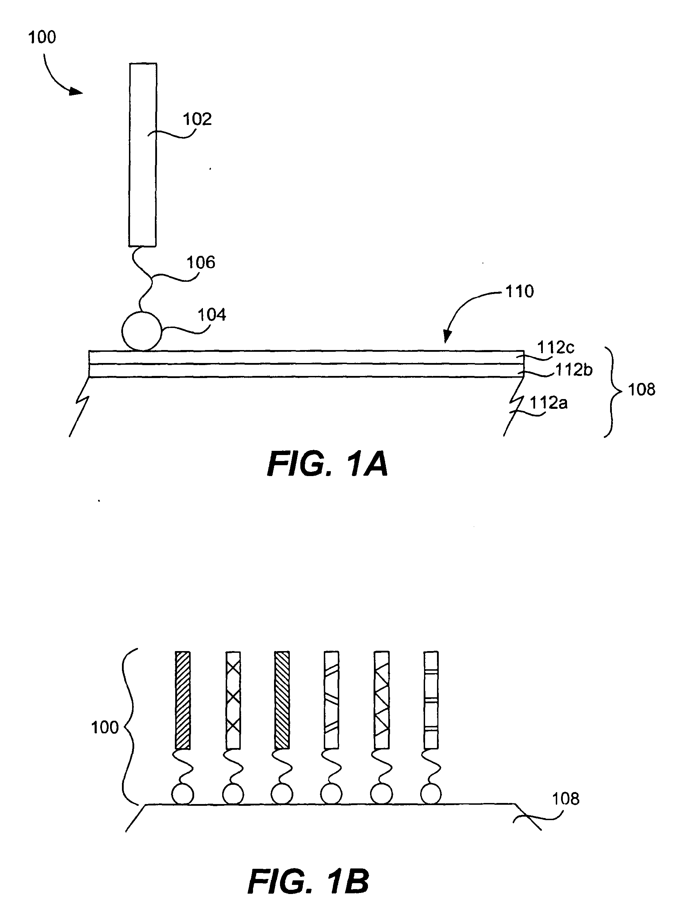

Figs. 1A and 1B provide a representation of one such array element and an array in accordance with the invention. In Fig. 1A, a protein-binding array element 100 includes three segments: a peptidomimetic segment 102, an anchor segment 104, and a linker segment 106. The array element is attached to a solid support or substrate 108 by a bond between its anchor segment 104 and the substrate surface 110. In some cases, the substrate may be composed of a plurality of layers 112a, 112b, 122c forming a laminate. In Fig. 1B, a protein-binding array includes a plurality of different array elements 100, such as illustrated in Fig. 1A, attached to a planar substrate 108. Each of these components of the array is described in greater detail separately below.

### A. Substrate

The solid support (Figs. 1A and 1B, element 108) employed in arrays in accordance with the present invention may vary greatly depending on the intended use of the product to be produced. The solid support may be any suitable material for binding the protein-binding agent that is also compatible with any analytical methods with which the array is to be used. Compatibility can be determined using methods and materials known to those having skill in the surface or materials chemistry arts. In one embodiment, the solid support comprises an impermeable, rigid material. Suitable materials include plastics, such as polymers, e.g. polyvinylchloride, polyethylene, polystyrenes, polyacrylates, polycarbonate and copolymers thereof, e.g., vinyl chloride/propylene polymer, vinyl chloride/vinyl acetate polymer, styrenic copolymers, and the like. Suitable materials also include glasses, such as those formed from quartz, or silicon; and metals (including alloys), e.g., gold, platinum, silver, copper, aluminum, titanium, chromium and the like.

As noted above, in many embodiments of interest, the support or substrate 108 will be a composite of two or more different layers of material, where the composition includes at least a base material, e.g., as represented by element 112a in Fig. 1A, and one or more surface coating materials, as represented by elements 112b and 112c in Fig. 1A. For example, one embodiment of interest includes a base material 112a, such as a glass, which is coated with a metallic layer 112b, e.g., gold or aluminum overcoated with silicon dioxide, or titanium overcoated with silicon dioxide, and a functionalized organic, e.g., amino-modified thiol or amino-modified silane, layer 112c. The planar solid support may be in the dimensions of a standard 3" x 1" microscope slide or in the shape of a 3" or 5" diameter circular wafer, for example. Other configurations will be apparent to those having skill in the surface or materials chemistry arts.

The solid support material or substrate may have a variety of different configurations, depending on the intended use of the material. Thus, in the broadest sense the support or base material may be in the form of a plate, sheet, cone, tube, well, bead, nanoparticle (e.g., about 5-100 nm), wafer, etc. In some embodiments, the base support material is one that has at least one substantially planar surface, e.g., as found on a plate, slide, sheet, disc, etc. In these embodiments, supports having an overall slide or plate configuration, such as a rectangular or disc configuration are employed.

In the planar, rectangular embodiments of the above-described slides, the length of the support will generally be at least about 1 cm and may be as great as about 40 cm or more, but will usually not exceed about 30 cm and may often not exceed about 20 cm. The width of support will generally be at least about 1 cm and may be as great as about 40 cm, but will usually not exceed about 30 cm and will often not exceed about 20 cm. The height of the support will generally range from about 0.01 mm to about 10 mm, depending at least in part on the material from which the rigid substrate is fabricated and the thickness of the material required to provide the requisite rigidity. Of particular interest in many embodiments are supports having the dimensions of a standard microscope slide. One typical substrate size is about 2.54 cm x 7.62 cm and about 1-2 mm thick. However, any suitable dimensions can be employed.

Where the support is a bead, nanoparticle or microparticle, the diameter of the support typically ranges from about 5 nm to about 1000µ, particularly from about 10nm to about 500 µ.

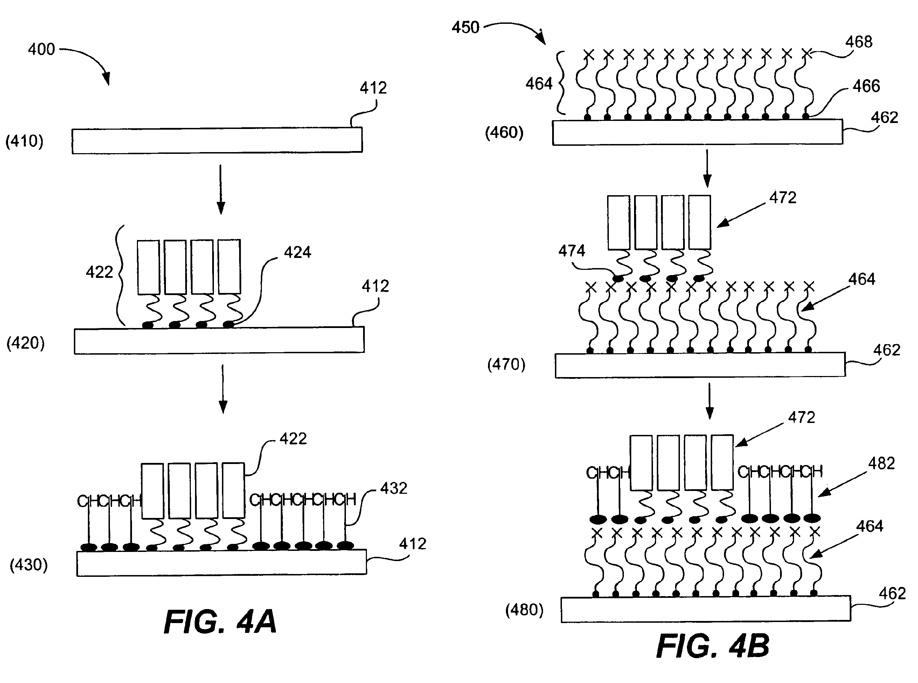

The protein-binding agents can either be attached directly to the inorganic solid surface of layers 112a or 112b (as illustrated and described in more detail below with reference to Fig. 4A) or attached using the functionalized organic layer of 112c (as illustrated and described in more detail below with reference to Fig. 4B). In the latter case, the non-substrate-bound termini of the organic molecules in the layer are functionalized with a reactive group that can attach to the anchoring group of a subsequently bound protein-binding agent. Suitable terminal reactive groups may be, for example, maleimide, hydrazide, aminooxy, an activated ester such as N-hydroxysuccinimide, anhydride, aldehyde, disulfide, thiol, azide, phosphine or avidin, streptavidin, neutravidin or other altered forms of the protein avidin that bind biotin, depending upon the anchor's functional group.

In those embodiments in which the surface of the base support material, such as glass, is coated with a thin layer of a metal, such as aluminum, gold, or titanium, the thickness of the metal layer will generally range from about 300 Å to about 10,000 Å, more particularly from about 750 Å to about 2,000 Å, and still more particularly from about 1,000 Å to about 1,500 Å. The metal layer may be deposited on the substrate surface using any suitable protocol, including e-beam deposition, vapor deposition, sputtering and the like, as are known to those of skill in the art. An adhesion metal layer may be present between the metal layer and the substrate, where adhesion metals of interest include titanium, chromium, and the like. When present, the adhesion metal layer will typically range in thickness between about 5 Å and about 100 Å, usually between about 25 Å and about 75 Å and in many embodiments will be about 50 Å. In some embodiments, the above-described adhesion layer can be a molecular adhesion layer. Examples of materials suitable for forming molecular adhesion layers in accordance with the present invention include mercaptopropyltriethyoxysilane, and other mercaptoalkoxysilanes, such as mercaptopropyltrimethoxysilane, mercaptopropyltrichlorosilane, or other chain lengths such as mrcaptohexyltriethoxysilane and other mercaptohexylalkoxysilanes, as are known in the art. Where the adhesion layer is a molecular adhesion layer, the thickness of the adhesion layer typically ranges from about 5 Å to about 50 Å.

The anchoring group of a protein-binding agent may be used to directly bond the protein-binding agent to an inorganic, e.g., metal or glass, substrate surface. For example, a thiol anchoring group may be used to bond directly to metals such as gold silver, copper or platinum without an intervening functionalized layer (e.g., maleimide/amine/thiol). Or, if the anchor group is an activated silane (i.e., modified to be reactive with other organic species, for example, hydrolyzable silane or modified silane that can condense with hydroxyls or other silanes to form siloxane bonds), e.g., chlorosilane or an alkoxysilane, the protein-binding agent can be directly bonded to glass or other oxide surfaces, such as titanium oxide, silicon oxide or aluminum oxide. Other activated silanes for this purpose include triethoxysilane, trichlorosilane, trimethoxysilane or other trialkoxysilanes. Within each of the classes, chain lengths may be from three atoms up to about eighteen atoms, for example.

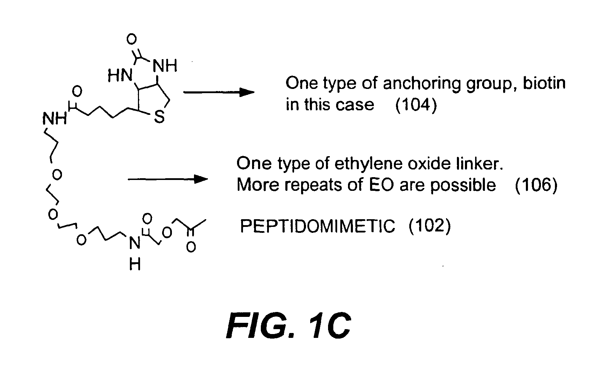

As will be described in more detail below, in the case of bonding of the protein-binding agent directly to a substrate's inorganic surface, the protein-binding agent's linking segment (between the peptidomimetic and the anchor) should be long enough to keep the peptidomimetic sufficiently far from the hard substrate surface that the surface does not interfere with protein binding occurring (subsequently) at the peptidomimetic. The length can be from about 2 atoms to about 200 atoms, or about 2 Angstroms to about 300 Angstroms, for example. A typical linking segment may have a backbone of between about 2 to about 30 atoms, preferably about 6 to about 12 atoms. The linking segment may be composed of, for example, suitably derivatized aliphatic chains (e.g., aminoalkanoic acids, such as aminohexanoic acid), ethylene oxides (e.g., such as shown in Fig. 1C), sulfoxides, or "non-binding" (orthogonal) short peptoid or peptide elements that remain constant for each element of the array (e.g., a glycine heptamer), or some combination of these components.

As noted above, the protein-binding agents may also be attached using a functionalized organic layer (Fig. 1A, element 112c), such as an amino-modified thiol (aminothiol) (for use with some metal substrate surfaces) or an amino-modified silane (aminosilane) (for use with glass, metallic or other oxide substrate surfaces). Where such an organic layer is used, the termini of the organic molecules of the layer are functionalized with a reactive group that can stably attach to the substrate surface at on end, and to the anchor segment of a subsequently-bound protein-binding agent in accordance with the present invention at another end. Suitable terminal groups include, for example, maleimide which can form a covalent bond upon reaction with a thiol presented in the anchor. A further advantage of such synthetic anchoring systems is their stability, conferring long shelf life. Other possible terminal groups on the substrate include hydrazide, which can react to form covalent bonds with aldehyde or ketone moieties in the anchor; aminooxy, which can react to form covalent bonds with ketone moieties in the anchor, aldehyde, disulfide, thiol, azide, phosphine.

In another implementation, proteins such as avidin, streptavidin, or other analogs may be used as a coating for the solid support. In this case, biotin is used as the anchoring moiety of the protein-binding agent to form a stable, non-covalent bonding complex with avidin on the surface. Without wishing to be bound by any particular theory of action, it is believed that this format displays the protein-binding agent in a particularly biocompatible environment with desirable distances between display molecules and their neighbors, and desirable distances between display molecules and the solid surface. Other protein-coatings can also be employed, provided they sufficiently bind a small molecule anchoring group. These may include anti-digoxigenin / digoxigenin, anti-dinitrophenol / dinitrophenol, or many other protein / small molecule pairs known in the art. Avidin / biotin is of particular value because of it's extremely stable binding interaction. In addition, substantial signal increases have been observed for biotin / avidin immobilization compared to other suitable immobilization techniques in accordance with the present invention, for example 1000X higher than thiol / maleimide. Suitable synthetic macromolecules may also be used as mimics of such protein spacers. These may include high molecular weight polymers such as polyethyleneimines, dendrimers, polyacrylic acids, polylysines and the like.

Of course, other suitable binding combinations of this character (namely, as noted above, sufficiently stable to maintain the bond during the processing to which the microarray is subjected under its normal operating conditions) are also possible. In addition, those having skill in the surface chemistry arts will understand that the above-listed anchoring groups on the peptoid can function as reactive groups on the surface, and the reactive surface groups can also function as a peptidomimetic anchoring groups.

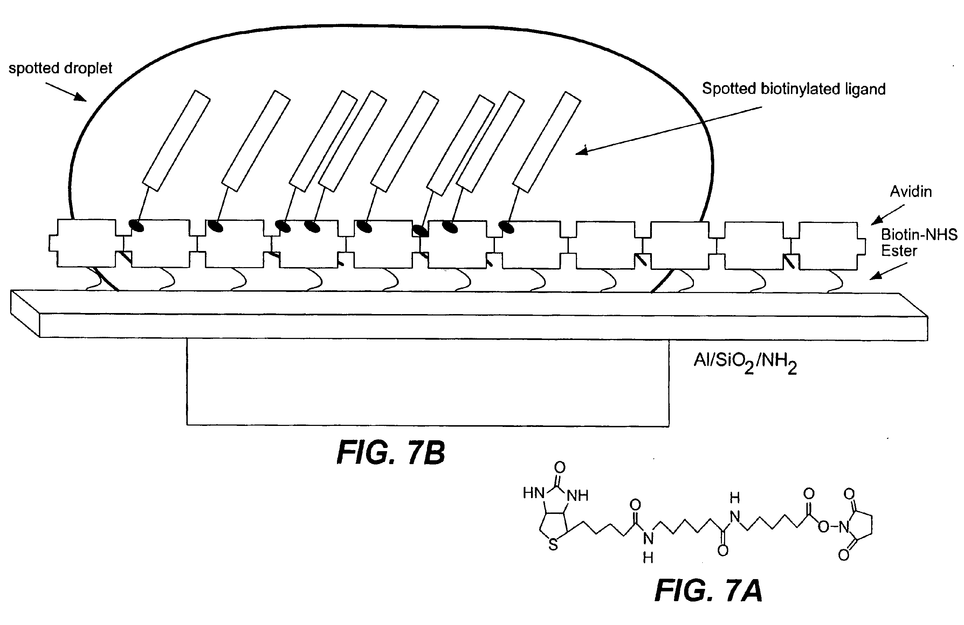

In one embodiment of the present invention, aluminum slides may be coated with a layer of silicon dioxide or silicon monoxide having a thickness of between about 500 Å to about 2,000 Å. The thickness is chosen to roughly correspond to 1/4 the wavelength of the emission or excitation light. A layer of an aminoalkyl trialkoxysilane, such as aminopropyl triethoxysilane (APS), trichlorosilane, trimethoxysilane, and any other trialkoxysilane is coated on the surface of the oxide. In addition, other amino-silanes could also be used, for example, compounds having longer alkyl groups, such as octyl, decyl, hexadecyl, etc., that may form more ordered silane layers as will be appreciated by those having skill in the surface chemistry arts. The thickness of this silane layer may be from about 3 Å to about 100 Å, more preferably about 5 Å to about 50 Å, even more preferably about 7 Å to about 20 Å. One suitable example is an APS layer that is about 7 Å thick. The amino-modified Al surfaces may be functionalized with a reactive group that will bind to the anchor functional group on a protein-binding agent. In one embodiment, the functional group may be maleimide. In another embodiment, the functional group may be a whole protein such as avidin. An implementation of an avidin-presenting substrate is described with reference to Figs. 7A and 7B in Example 3, below.

In another embodiment, a gold-surfaced substrate slide (or a slide surfaced with any metal capable of forming metal-thiol bonds, such as Ag, Pt, or Cu) may be coated with an aminothiol layer that is functionalized with a group that will bind to the anchor functional group on a protein-binding agent, such as maleimide. However, the anchor functional group and the surface-bound reactive group should be chosen to have orthogonal reactivities. As noted above, these anchor and substrate-bound groups can be interchanged as will be readily understood by those skilled in the art.

In general, the functionalized organic layer 112c is characterized by having a substantially uniform hydrophilic surface. By uniform is meant that the surface of the layer includes substantially no irregularities, such as gaps, pinholes, etc. The thickness of the layer 112c may vary considerably, where the thickness may be less than about 5,000Å, usually less than about 2,000 Å, but can be much lower, particularly from about 5 Å to about 100 Å, or between about 20 Å to about 50 Å.

In at least those embodiments where the support material includes a metallic layer beneath a functionalized organic layer, e.g., where the support material is an amino-modified thiol layer on a gold coated microscope slide as described above, the thickness of the organic layer is chosen such that the layer 112c is at least sufficiently thick to separate any fluorescently labeled moiety that may be present to the surface a sufficient distance from the metallic layer on the substrate surface such that significant signal quenching (i.e., signal quenching of sufficient magnitude to effectively preclude meaningful detection of the signal) does not occur. In these embodiments, the functionalized organic layer may have a thickness that is at least about 30 Å, usually at least about 50 Å and more usually at least about 100 Å. Alternatively, in cases where quenching does occur, the slide may be treated with a high salt solution such as 0.75 M sodium chloride, 0.085 M sodium citrate (*see. e.g.,* PCT Publication No. WO 01/01142).

In those embodiments where the array is employed with fluorescently labeled target, as described in greater detail below, the thickness of the functionalized amino-modified thiol layer may be chosen to provide for maximum amplification of the emitted and reflected signals. See e.g., U.S. Patent No. 5,055,265. In such embodiments, the thickness of the organic layer will be about ¼ of the wavelength of the emitted light from the label. While the exact thickness of the organic layer will vary depending on the particular label with which it is to be employed, the thickness generally ranges from about 50 Å to about 300 Å, usually from about 100 Å to about 200 Å and more usually from about 125 Å to about 150Å.

In one embodiment, the functionalized amino-modified thiol layer 112c includes at least one self-assembled monolayer (SAM). As such, the functionalized organic layer may include one or more different self-assembled monolayers grafted sequentially onto each other, where when the layer includes more than one self-assembled monolayer *(see. e.g.,* PCT Publication No. WO 01/01142).

### B. Protein-Binding Agents

As noted above, the protein-binding agents of the present invention are composed of three segments: a peptidomimetic, an anchor, and a linker connecting the peptidomimetic and the anchor.

A "peptidomimetic" as used herein refers to nonpeptide synthetic polymers or oligomers that detectably interact with proteins or receptors in a manner analogous to protein-protein or protein-peptide physical chemical interactions under assay conditions. Peptidomimetics are generally protease-resistant, and include, for example, oligomeric species such as peptoids, beta-peptides, and others as described in A.E. Barron & R.N. Zuckermann, *Bioinspired polymeric materials: in-between proteins and plastics,* Curr Opin Chem Biol 1999, 3(6):681-7 and K Kirchenbaum, R.N. Zuckermann and K.A. Dill, *Designing polymers the mimic biomolecules,* Curr Opin Chem Biol 1999 9(4):530-5, and constrained cyclic molecules such as cyclic peptides and heterocyles. In some cases, the peptidomimetic may also mimic the folding of natural proteins. Peptidomimetics in accordance with the present invention comprise generally no more than about 500 mers, more particularly no more than about 100 mers, typically no more than about 50 mers, and usually about 10 to about 20 mers, more particularly about 12 to about 16 mers. Given the parameters provided herein, one of skill in the art would be able to choose an appropriate peptidomimetic length for a given application without undue experimentation.

Examples of peptidomimetic libraries and their design and synthesis may be found in Fahad Al-Obeidi, et al., *Peptide and Peptidomimetic Libraries,* Molecular Biotechnology 1998, 9: 205-223; Victor J. Hruby et al., *Synthesis of oligopeptide and peptidominetic libraries,* Curr Opin Chem Biol 1997, 1:114-119; and Amy S. Ripka et al., *Peptidomimetic Design,* Curr Opin Chem Biol 1998, 2:441-452.

In a preferred embodiment of the present invention, the peptidomimetic segment of the protein-binding agent is a peptoid. The term "peptoid" as used herein refers to polymers comprising N^{α}-substituted amides as described in U.S. Patents Nos. 5,831,005, 5,877,278, 5,977,301, 5,871,387, 5,986,695, 5,719,049 and 5,977,301, 6,211,468; and PCT Publications Serial Nos. 96/15143 and 93/09117.

The anchoring segment provides for the stable attachment of the array element to the solid surface. This functional group is chosen to be reactive towards a complementary group that is displayed on the solid surface and orthogonal to (*i.e*., substantially non-reactive with) sidechains present in the ligand. An important feature of the anchoring group is that its reaction to the surface be sufficiently facile so that it is complete within the average lifetime of a droplet that is deposited by the robotic array spotter onto the surface. For example, if the surface displays a maleimide, a suitable anchoring group is a thiol and approximately 15-20 minutes at about 60% humidity are required for completion of the binding reaction before the approximately 10 nL drop evaporates. Of course, droplet lifetime varies with temperature, humidity and other conditions, allowing more or less time for the reaction to take place. As noted above, other surface display/anchor combinations are possible, including a hydrazide surface group with an aldehyde or ketone anchoring group. Or, the anchoring group can also be a biotin molecule, that will attach strongly (yet non-covalently) to a surface that displays an avidin protein.

The anchoring group may be attached to the peptidomimetic at either end (in the case of a peptoid, at either the C- or N-terminus). It can be attached either as a submonomer (e.g., a substituted amine), or as a modification of a peptidomimetic (e.g., peptoid) side chain after synthesis. Alternatively, in another example, the anchoring group may be present as a linker connecting the peptoid to a beaded solid support upon which it is synthesized. This linker may be cleaved from the resin along with the peptoid, to provide a readily available anchoring group.

The linking segment of the protein-binding agent molecule is chosen to provide for separation between the solid surface and the peptidomimetic segment sufficient to facilitate interaction between the peptidomimetic and the components of the analyte solution (solution with which the microarray will be contacted), for example by providing separation between the substrate surface and the peptidomimetic so that the surface does not interfere with protein binding occurring (subsequently) at the peptidomimetic access for a protein binding site to the peptidomimetic ligand displayed on the surface, especially for proteins with deep binding pockets. The linker may also serve to separate the peptidomimetics on the surface from each other, thereby mitigating possible steric hindrance between the ligand and protein binding pocket. A typical linking segment may have a backbone of between about 2 to about 200 atoms, preferably about 6 to about 30 atoms. The linking segment may be composed of, for example, aliphatic chains (e.g., aminoalkanoic acids, such as aminohexanoic acid), ethylene oxides, sulfoxides, or "non-binding" ("orthogonal") short peptoid or peptide elements that remain constant for each element of the array, or some combination of these components. In one embodiment, a 2-carbon linker may be used. In another embodiment, three ethylene oxides may be used. An example of a non-binding short peptoid is a 2-mer to 12-mer, for example a 4-mer of methoxyethyl side chains that remain constant for each protein binding agent, while the peptidomimetic segment is variable. A suitable orthogonal peptide linker is a 2-mer to 12-mer, for example a 5-mer of glycine.

In some embodiments, where an organic layer is present on the substrate surface, a spacer functionality may be built into the organic layer. In such cases, the spacer in the substrate surface layer and the linker in the protein-binding agent may collectively contribute to the desired spacing of the peptidomimetic from the hard substrate surface. Further, the mitigation of steric hindrance may also be achieved by the selection of a surface coating that presents a particularly biocompatible format, such as an avidin protein (coupled with a biotin anchoring group on the protein-binding agent).

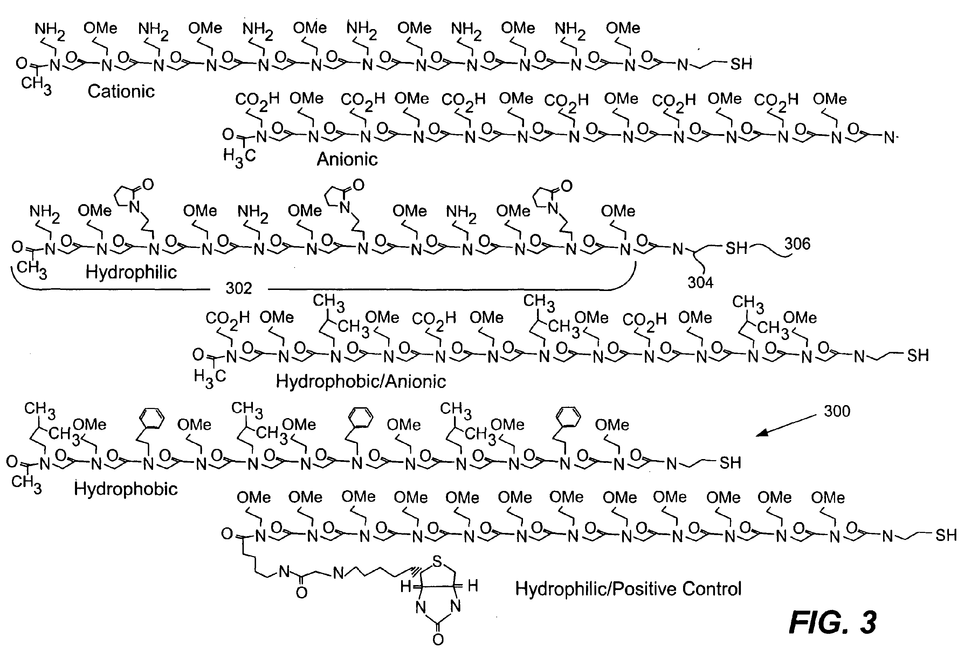

Fig. 3 illustrates an example of a small set of array elements 300 wherein the peptidomimetic protein-binding segments 302 are 12-mer peptoids, the linker 304 is a short aliphatic chain, and the anchoring group 306 is a thiol. The peptidomimetic segments depicted in Fig. 3 illustrate a subset of the range of affinity properties achievable using peptoids as the peptidomimetic segment, in accordance with one embodiment of the present invention

As mentioned above, the number of different types of binding agents present on the surface of the array is at least two. By "different", it is meant that the sequence of monomeric units between two different peptidomimetics of two different protein-binding agents is not the same. While the number of different species of protein-binding agents present on the surface of the array is at least 2, at least about 10, at least about 50, or at least about 100, it is typically much higher, generally being at least about 1,000 usually at least about 5,000 and more usually at least about 10,000. The number may be as high as 500,000 or higher, but typically does not exceed about 100,000 and usually does not exceed about 50,000.

The surface regions surrounding the protein-binding array elements may be modified so as to minimize background non-specific binding of proteins, allowing complex samples (e.g., lysates or serum) to be examined in a single step. The surface may be blocked chemically with hydrophilic termini such as alcohols, carbohydrates, amino acids, sulfoxides, acrylamides, or ethers. Examples of alcohol terminal groups are, for example, mercaptoethanol, mercaptohexanol, mercapto-octanol, in the case of a maleimide-treated surface. These block the unreacted maleimides on the surface. The surface can also be blocked using proteins such as solutions of 1% - 10% bovine serum albumin ("BSA") or human serum albumin ("HSA") in phosphate buffered saline, or 1% - 10% non-fat dry milk or 1% - 10% casein, gelatin or other suitable blocking protein. In some cases, the addition of detergents to the blocking protein solution is advantageous. For example, the addition os 0.01% - 0.5% Tween-20 or Triton X-100 (particularly 0.05%), 0.1 - 2% SDS as are well know in the assay development or surface chemistry arts.

### C. General Features of the Array

Typically, the array is characterized by having a plurality of protein-binding agent spots on a solid substrate, where each spot is characterized by having one or more, usually a plurality, of identical binding agents bound to the support surface. The number of distinct spots on the surface of the array may or may not be the same as the number of different protein-binding agents on the array, e.g., the same protein-binding agent may be presented in two or more spots on the array surface. In one embodiment, each protein-binding agent is presented in duplicate in the array. Depending on the nature of the binding agents, the size of the support surface, the methods of fabrication and the intended use of the array, the number of distinct spots on the array surface may vary greatly. Where the support surface has the dimensions of a standard microscope slide (about 3" x 1"), the number of spots on the support surface will typically be at least about 3,000, usually at least about 6,000 and more usually at least about 10,000 - 50,000. The number may be as high as 100,000 or higher, but typically does not exceed about 75,000 and usually does not exceed about 50,000.

The diameter of each spot will typically range from about 100 µm to about 300 µm, usually from about 200 µm to about 300 µm. The space between any two given spots will generally be between about 1 µm and about 50 µm. The density of the spots generally ranges from about 1 to about 5,000 spots/cm², usually from about 100 to 2,000 spots/cm². Typically, the spots are arranged across the surface of the spacer layer in the form of a pattern. The pattern may be in the form of organized rows and columns of spots, e.g., a grid of spots, across the substrate surface, a series of curvilinear rows across the substrate surface, e.g., a series of concentric circles or semi-circles of spots, and the like. To further increase density, the spots may also be hexagonally arranged. Still other arrangements of spots are within the scope of the present invention.

### 2. Methods of Making the Protein-Binding Agent Arrays of the Subject Invention

The arrays of the subject invention may be prepared using any convenient protocol. One protocol of interest involves 1) the procurement of a solid support having a surface activated for binding of a protein-binding agent; and 2) contact of two or more different protein-binding agents with the support surface under conditions such that the protein-binding agents become stably associated with the support surface.

### A. Solid Support Fabrication

The solid support may be fabricated using any convenient methodology, which methodology will vary depending the particular nature of the solid support. In accordance with one embodiment of the invention a glass support is coated with a layer of metal, e.g., aluminum or gold. To prepare a solid support of glass coated with a metal layer, the surface of the glass is coated with a thin layer of the metal, e.g., gold, silver, platinum, copper, titanium, or aluminum, etc. in a thickness as described above. The metal layer may be deposited on the substrate surface using any convenient protocol, where suitable protocols include e-beam deposition, vapor deposition, sputtering, and the like, and are known to those of skill in the art. See e.g., Moteshari et al., J. Am. Chem. Soc. (1998) 120:1328-1336; Bain et al., J. Am. Chem. Soc. (1989) 111:7155-7164; Lee et al. Langmuir (1998) 14:6419-6423; Folkers et al., Langmuir (1992) 8:1330-1341. Where convenient, an adhesion metal layer may be present between the metal layer and the substrate, where adhesion metals of interest include titanium, chromium, and the like, deposited in a thickness as described above. In addition, oxide overlayers such as silicon dioxide or silicon monoxide may be deposited by e-beam or sputtering deposition on top of the metallic layer.

In one example, following preparation of a gold substrate, if the protein-binding agent's anchoring group is a thiol and the linking group is sufficiently long (as explained above), arrays in accordance with the present invention can be formed by spotting thiol-displaying protein-binding agents onto bare, clean gold. The gold surface of the substrate may be cleaned using a chromic acid cleaning solution (e.g., chromium oxide or sodium dichromate in sulfuric acid, for example Nochromix, available from Fisher (50-80% Sodium dichromate in 12 N sulfuric acid)) and rinsed with HPLC-grade water. This has the advantage of reducing the number of surface functionalization steps.

In another embodiment, where the protein-binding agent's anchoring group is a functionalized modified silane (e.g., mono-, di- or tri- functional silanes, such as chlorosilane, an alkoxysilane, dichloro or dialkoxy silane, or trichloro or trialkoxy silane), the anchoring group can be attached directly to oxide-containing surfaces such as glass, titanium oxide or aluminum oxide. Arrays in accordance with these embodiments of the present invention can be formed by spotting active silane-displaying protein-binding agents onto the oxidized substrate surface. A glass substrate has surface hydroxyls available for this binding. Metal substrate surfaces may be oxidized, for instance by thermal or chemical treatment. For example, aluminum may be oxidized electrochemically, thermally or chemically (e.g., with H₂O₂), as is well known in the art. In addition, a silicon dioxide or titanium oxide layer can be deposited on the aluminum. An oxide may also be present as a native thin layer, such as occurs with aluminum or silicon. This native oxide may then be derivatized by the amino-silane.

In still another embodiment of the invention, a metal, e.g., gold or aluminum, substrate surface is first functionalized with functionalized organic molecules that form ordered monolayers. The termini of the organic molecules are functionalized with a reactive group that can attach to a suitable anchoring group of a protein-binding agent. Suitable terminal groups may be, for example, maleimide, hydrazide, aminooxy, an activated ester such as N-hydroxysuccinimide, anhydride, aldehyde, disulfide, thiol, azide, phosphine or avidin, depending upon the anchor's functional group.

In one embodiment the functionalized organic molecules are amino-modified thiol molecules. In order to functionalize the metal surface with the thiol molecules, the substrate having the metal surface may be dipped in a solution of the amino-modified thiol; the amino-modified thiol solution then may be deposited onto the surface of the substrate. Other convenient protocols may be employed. Typically, the gold substrate is immersed into the amino-modified thiol solution under conditions and for a sufficient period for the amino-modified thiol molecules to assemble into a monolayer on the substrate surface. The temperature at which contact is carried out typically ranges from about 10°C to about 100°C, usually from about 15°C to about 80°C. Contact is maintained for a sufficient period of time for the self-assembled monolayer to form on the gold surface, where contact is typically maintained for at least about 20 minutes, usually at least about 4 hours, and more usually at least about 16 hours.

Alternatively, the amino thiol can also be deposited by vapor deposition in a vacuum oven or by spin coating. For example, a 1 - 10% (e.g., 5%) solution of thiol in a volatile solvent such as isopropanol, methanol, THF may be prepared. The slides may be spun at about 1000 - 8000 rpm (e.g., 5000 rpm) to provide an even deposition of the thiol. Then, a heterobifunctional molecule (e.g., succinimidyl 4-(N-maleimidomethyl)-cyclohexane-1-carboxylate (SMCC) or LC (long-chain) - SMCC) that is an activated ester on one end and a maleimide on the other is contacted with the amino group to create the maleimide-terminated surface. Other heterobifunctional cross-linkers could also be used with different length spacers (e.g., a long ethylene oxide spacer (e.g., 2-4 units) between an NHS ester on one end, and a maleimide on the other end. As noted above, the spacer on the substrate serve the same purpose as and the "linker" in the protein-binding agent.

In another embodiment, Aluminum slides may be used. Aluminum metal may be deposited by e-beam deposition onto a clean glass substrate. The aluminum is then overcoated by silicon dioxide (SiO₂) or silicon monoxide in a thickness that is the same or thinner than 1/4 the wavelength of the emission or excitation light. Oxide thicknesses of about 600 to 1000 Angstroms may be used as these eliminate the need for thinning the oxide prior to performing binding experiments. The aluminum / oxide surface may be treated with a amino-modified silane. For example, aluminum slides freshly coated with a 800 - 1,400 Angstrom layer of silicon dioxide, may be dipped immediately into a bath of 3% - 40% aminopropyl triethoxysilane in isopropanol, that has been previously filtered through an 0.2 uM filter membrane, and silanized for up to 1 hour, followed by rinsing and drying. Alternatively, aluminum slides coated with silane (APS) are available from Amersham-Pharmacia, Amersham, England (and described in International Patent Application No. WO 98/53304). In some cases, such commercially available slides may require thinning of the oxide layer to between about 200 Å and about 1,000 Å, more particularly between about 900 Å and about 1,000 Å, prior to performing a binding experiment to improve signal-to-noise ratios. The final amino-modified Al surfaces may be functionalized with SMCC to render a surface that presents maleimide functional groups. Here again, the silane may also be vapor deposited or spin coated, as described above. For example, a 1 - 10% (e.g., 5%) solution of silane in a volatile solvent such as isopropanol, methanol, THF may be prepared. The slides may be spun at 1,000 - 8,000 rpm (e.g., 5,000 rpm) to provide an even deposition of the silane. Then, a heterobifunctional molecule (e.g., succinimidyl 4-(N-maleimidomethyl)-cyclohexane-1-carboxylate (SMCC or LC-SMCC)) that is an activated ester on one end and a maleimide on the other is contacted with the amino group to create the maleimide-terminated surface. Other heterobifunctional cross-linkers could also be used with different length spacers (e.g., a long ethylene oxide spacer (e.g., 2 - 4 units) between an NHS ester on one end, and a biotin or maleimide on the other end. As noted above, the spacer on the substrate serve the same purpose as and the "linker" in the protein-binding agent.

Further in accordance with this embodiment, it has been found that amplification of the fluorescent signal used in assays conducted with microarrays in accordance with the present invention may be enhanced by etching the commercially available functionalized and spotted aluminum slides (Amersham) to reduce the oxide layer thickness to about 200 Å to about 900 Å, preferably about 500 Å. For example, an etch solution of about 0.1 - 0.2 % SDS / 5X SSC (0.75 M NaCI/0.085 M sodium citrate) and optionally 5mM EDTA may be applied at about 50 - 80 degrees, preferably about 60 °C for about two to four hours. As noted above, manual deposition of a silicon dioxide layer of a suitable thickness may eliminate the need for the thinning of the slide.

As noted above, where the substrate surface has an organic self-assembled monolayer, following self assembly of the initial monolayer, one or more additional monolayers may be grafted onto the initial monolayer, where the additional layer(s) are made up of molecules, e.g. alkyls, having functionalities that provide for their covalent attachment to the surface functionalities of the initially deposited monolayer, e.g. amino functionalities where the initially deposited monolayer is characterized by the presence of carboxy functionalities. Alternatively, multi-component spacers can be constructed prior to attachment to the surface.

### B. Synthesis of the Protein-Binding Agents

As noted above, protein-binding agents in accordance with the present invention are composed of three segments: a peptidomimetic, an anchor, and a linker connecting the peptidomimetic and the anchor.

Peptidomimetics may have a variety of nonpeptide polymeric structures and characteristics as long as they can mimic the biological effects of natural peptides. References to peptidomimetic structures have been provided above. In one embodiment of the present invention, the peptidomimetic segment of the protein-binding agent is a peptoid.

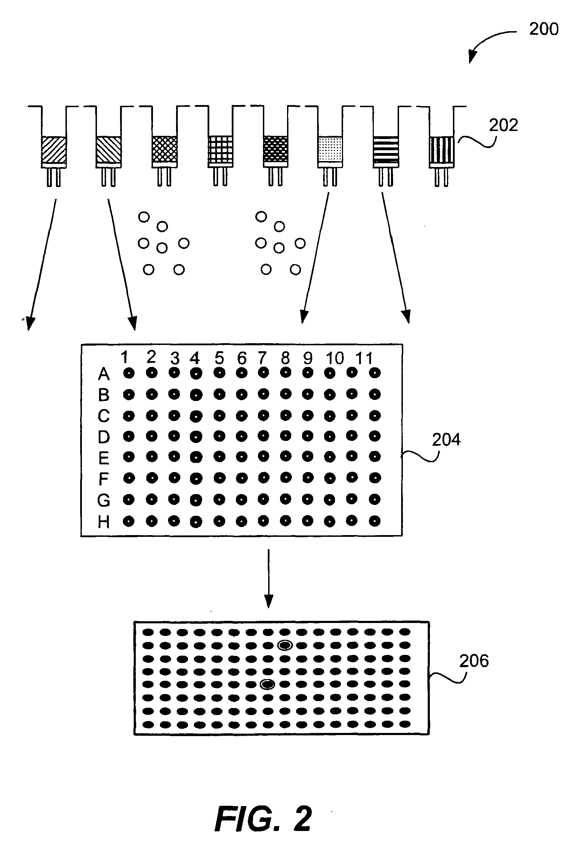

Libraries of peptoids may be synthesized using robotic solid-phase synthesis techniques, such as those developed by Chiron Corporation of Emeryville, CA. The diversity of the library can be controlled by the nature and arrangement of the amine submonomers (bromoacetic acid and substituted amines) used in the peptoid synthesis, as described in above-incorporated U.S. patent documents. As illustrated in Fig. 2, libraries may be prepared according to a process 200 using mix and split synthesis 202, or parallel synthesis, such that there is a multiplicity of peptoids synthesized, but only one species of peptoid on a given bead (i.e., each bead has one unique compound, repeated many times). The amount of compound per bead may range from about 1-100 nanomoles, with 20 - 40 nanomoles being most typical. About 40 nanomoles per bead are commonly obtained. The synthesized peptoids, still bound to resin, may then be distributed to 96-well plates (or other multi-well plates, such as 384-well or 1024-well plates) 204 using, for example, bead picking technology described in the just-referenced U.S. Patent application. The use of 'big bead' resin (having a diameter of about 500 µ) allows distribution of one bead per well.

The peptoids may then be then dried, cleaved from the resin (e.g., with about 20 - 95% TFA in dichloromethane; 95% being typical), re-dissolved in acetonitrile/water, filtered or centrifuged, dried and re-dissolved in 50% DMSO / 50% Buffer. Other solvent / buffer systems can also be used such as 50% DMF or 50% NMP with the remainder being PBS, TBS, Borate, Tris-HCl, etc. In some cases, a reducing agent such as tris-carboxyethylphosphine (TCEP) may also be added to the wells of the spotting plate to inhibit oxidation of the thiols on the peptoids so that they retain their reactivity toward maleimides on the substrate surface. In cases where TCEP is added to the wells, phosphate buffers such as PBS are to be avoided, and buffers such as TBS would be preferred. This final solution is ready for spotting.

In one embodiment of the present invention, the concentration of peptoids in the wells of the spotting plate should be in the range of about 0.5 - 2 mM, where 2 mM is preferred. For spotting onto the prepared planar solid support 206, the peptoids are filtered or centrifuged, dried, and resuspended, as described in further detail below. It is useful for the material spotted on the slide be in an excess with respect to the amount of probe in solution to be applied to the microarray. This is an amount that gives a saturating fluorescence signal, so that changes in signal intensity are due substantially to protein levels.

The anchoring and linker groups may be attached to the peptoid at either the C- or N-terminus. They can be attached either as a submonomer (e.g., as described in U.S. Patent No. 5,877,278) during the peptoid synthesis as described above in the patent documents, or with in situ activated amino acid coupling steps, as a modification of the peptoid after synthesis, according to procedures known to those of skill in the art.

For N-terminal attachment, the peptoid may be synthesized on a resin and then the linker and the anchor groups may be added before the entire molecule is cleaved. For example, peptoids may be prepared on Rink amide polystyrene resin as illustrated and described in co-pending Application No. 09/704,422. The synthesis procedure is also reported in Figliozzi, G. M., Goldsmith, R., Ng, S. C., Banville, S. C., Zuckermann, R N. *Methods Enzymol.* 1996, 267:437-447. Also, before cleavage, one or more (e.g., two to four, preferably four) submonomer hydrophilic linker groups (e.g., methoxyethylamine) may be added to the peptoid N-terminus. Then, a trityl-protected cysteamine is added in order to provide a thiol anchoring group. The peptoid with attached linker and anchoring groups may then be cleaved from the resin, for example using 95 % TFA (v/v) in dichloromethane (CH₂Cl₂). The resulting solution is then ready for application (spotting) onto microarray slides in accordance with the present invention.

In another embodiment, an FMOC-protected beta-alanine is attached to the N-terminus of the peptoid by in situ activation with Hobt and DIC, as is known to those skilled in the arts of peptide synthesis. The beta alanine functions as an adaptor molecule between the peptoid and peptide linker. After attachment of beta-alanine, an FMOC-protected amino acid on a peptide (e.g., glycolic) linker, for example, is attached to the N-terminus of the beta-alanine. Finally, biotin is added to the N-terminus by peptide coupling.

The thiol or biotin anchoring group can also be attached to the end of the peptoid at the C-terminus. For example, 4-(diphenylhydroxymethyl)benzoic acid (available from Fluka) is treated with cystamine hydrochloride in the presence of an acid catalyst. Next the resulting amine is protected as the *N*-(9-flurenylmethoxycarbonyl) (*N*-FMOC) derivative, and the resulting FMOC-NH-CH₂CH₂-S-Tr-COOH is coupled to aminomethyl-Big Beads (400-500 microns, Polymer Labs). The peptoid is synthesized on the deprotected amine as described above, and treatment with TFA results in cleavage of the thiol-modified peptoid from the resin, while leaving the trityl protecting group on the resin.

### C. Contacting the Solid Support with Protein-Binding Agents

Following preparation of the substrate and protein-binding agents, as described above, two or more different protein-binding agents of interest that are to be bound to the surface to produce the array are contacted with the functionalized, or otherwise prepared (cleaned, oxidized, etc.) substrate surface. By contact is meant that the binding agents are brought into proximity with the surface such that they become substantially stably attached or bound to the surface of the substrate layer.

In contacting the binding agents with the substrate surface, any convenient means for contacting the surface with the binding agents which results in the desired pattern of binding agent spots, as described above, maybe employed ,*e.g*., by spotting. As mentioned above, the term contact is used herein to refer to any method that brings the binding agent within close proximity of the support surface. Generally, an aqueous solution (e.g. water, water/organic solvent (such as 50/50 water/DMSO, or the like) of the binding agent is employed during contact where the solution may comprise one or more components in addition to water and the binding agent, e.g., buffering agents, salts, and the like. Other systems include 50/50 NMP/TBS, DMF/TBS, NMP/PBS, DMF/PBS, or all these solvents together with water. The 50/50 mix can also be adjusted. For example, when a higher percentage of the aqueous solution is used, the drop sizes can be smaller because of the higher surface tension of the solution. Drop size (and therefore density) may be controlled to some extent in this manner. Typically, contact is achieved by depositing solutions of the different binding agents onto discrete locations of the support surface, such that each different type of binding agent is deposited onto its own unique location on the substrate surface.

The binding agents may be deposited onto the support surface using any convenient means, *e.g.*, by pipetting. A number of devices and protocols have been developed for depositing aqueous solutions onto precise locations of a support surface and may be employed in the present methods. Such devices include "ink-jet" printing devices, mechanical deposition or pipetting devices and the like. See e.g., U.S. Patent Nos. 4,877,745; 5,338,688; 5,474,796; 5,449,754; 5,658,802; 5,700,637; and 5,807,552. Robotic devices for precisely depositing aqueous volumes onto discrete locations of a support surface, i.e., arrayers, are also commercially available from a number of vendors, including: Genetic Microsystems; Molecular Dynamics; Cartesian Technologies; Beecher Instruments; Genomic Solutions; and BioRobotics. Alternatively, bubble jet technology recently described, by Okamoto, Suzuki and Yamamoto, *Nature Biotechnology,* vol. 18 (April, 2000), 438, may be used.

As noted above, an important feature of a process in accordance with the present invention is that the reaction between the anchoring group on the protein-binding agent and the substrate surface must be sufficiently facile so that it is complete within the average lifetime of a droplet that is deposited by the robotic array spotter onto the surface. For example, if the surface is functionalized and displays a maleimide, a suitable anchoring group is a thiol and approximately 15-20 minutes at about 60% humidity are required for completion of the binding reaction. As noted above, other surface display/anchor combinations are possible, including those forming stable, yet non-covalent bonds, such as avidin and biotin.

### D. Blocking the Chip

After spotting of the peptoid library onto the array substrate (chip), the remaining, uncoated surface of the chip may be functionalized with a molecule that displays a hydrophilic terminus. These hydrophilic termini are anticipated to reduce or eliminate non-specific binding of proteins in the complex mixture. The hydrophilic portion may consist of alcohols, sulfoxide, carbohydrates, acrylamides, with hydrophilic termini such as alcohols, carbohydrates, amino acids, sulfoxides, acrylamides, and ethers or other low-protein binding group. The hydrophilic display molecule is anchored to the chip in the same manner as the peptoid that has already been spotted. For example, chips spotted with the peptoid library may be chemically blocked with cysteine, mercaptoethanol or other suitable hydrophilic thiol. The chips may also be blocked with protein such as 2% BSA / PBS, 10% non-fat dry milk or 1% casein for at least 1 hour, rinsed with water and dried. Other possible blocking agents are noted above. The blocking agents may be applied to the chips in ways well known to those of skill in the art, such as by dipping the chips in a solution of a blocking agent, by painting the surface of the chips with a blocking agent solution, or by spin-coating.

### E. Summary

Figs. 4A and 4B briefly illustrate processes for making protein-binding agent arrays for some embodiments of the invention in accordance with the procedures described above. In Fig. 4A, a process (400) for making an array in which protein-binding agents are bound directly to the inorganic surface of a bare planar substrate is depicted. A planar substrate 412 with a gold or aluminum surface is provided (410). The surface is prepared for binding (cleaned) as described above. Protein-binding agents 422 with a thiol anchoring group 434 are spotted onto the substrate 412 (420). Once binding of the protein-binding agents is complete, a blocking agent 432, namely a hydrophilic group, such as an alcohol, or a protein is applied to the surface of the substrate 412 where no protein-binding agent 422 is bound (430).

In Fig. 4B, a process (450) for making an array in which protein-binding agents are bound to the surface of a planar substrate via an organic surface layer is depicted. A planar substrate 462 with a gold or aluminum surface is provided (460). The surface is prepared for binding by applying a functionalized amino-modified thiol or amino modified silane layer 464, as described above. The layer 464 includes a thiol or silane functionality 466 which binds to the gold or aluminum surface of the substrate 462 and a binding functionality 468, such as maleimide, for a subsequently bound protein-binding agent. Protein-binding agents 472 with anchor group functionality 474 complementary to the substrate surface layer binding functionality 468 are spotted onto the substrate (470). Once binding of the protein-binding agents is complete, a blocking agent 482, namely a hydrophilic group, such as an alcohol, or a protein is applied to the surface of the substrate where no protein-binding agent 472 is bound (480).

### 3. Methods of Using the Protein-Binding Agent Arrays of the Subject Invention

The subject arrays find use in a variety of different applications in which binding events between the surface bound binding agents of the array and analyte(s) of interest in a test sample are detected. In other words, the arrays of the subject invention find use in binding assays. In such applications, the support bound binding agent generally acts as a "target" for the analyte "probe" in the test sample. The analyte probe is typically labeled, e.g., where the label may be a directly detectable label (e.g., radioactive isotope, fluorescent label, chemiluminescent label, etc.) or an indirectly detectable label (e.g., member of a signal producing system, such as a ligand for a labeled antibody, where the label may be enzymatic which converts a substrate to a chromogenic product, etc., where the labeled antibody may be a secondary labeled antibody) so that binding events may be readily detected.

In particular, arrays in accordance with the present invention are useful in performing proteomic analyses of complex protein samples. As used herein, proteomics is the separation and/or quantitation and/or identification of one or more proteins in a sample. The sample may be derived from a cell (e.g., the cell's cytosol, membrane or extra-cellular proteins), tissues (e.g., dissected or laser-microdissected), body fluids (such as urine, blood spinal fluid) or any other sample containing proteins. The results of such separation/quantitation/identification may produce novel protein targets for drug screening, proteins for diagnostics, or novel synthetic ligands for assays or protein purification. The arrays may very effectively be used in differential protein binding assays. For example, two (or more)-color fluorescent labeling of complex protein mixtures, and the analysis of differential protein binding to the array by fluorescence imaging may be conducted. As described below, the arrays may be used in conjunction with other techniques to identify, sequence and structurally characterize differentially expressed proteins or peptides of interest. The arrays may be run in parallel with DNA arrays and the differential binding results compared to identify correlations between gene activity and protein expression. Also, mixed arrays, wherein the molecules making up an array includes antibodies, etc. may be prepared and used to conduct binding assays.

A variety of techniques can be used to conduct differential binding assays using arrays in accordance with the present invention ("proteomic microarrays"). Some of these techniques, as used in embodiments of the present invention, are described below:

### A. Protein Labeling

Complex protein samples are labeled using standard techniques, many of which have been developed for 2-D gel analysis of protein mixtures. For example, sample A may be labeled with an amine reactive Cyanine 3 dye ("Cy 3") (λₑₓ = 550nm / λₑₘ = 570nm), and sample B is labeled with an amine reactive Cyanine 5 dye (Cy 5) (λₑₓ = 650 / λₑₘ = 670 nm) (dye reagents available from Amersham-Pharmacia). Samples A and B may be, for example, from normal or diseased, treated or untreated, etc., tissues or cell lines, respectively. The unreacted dye may be separated from the labeled protein using standard methods such as gel filtration, dialysis, etc. Of course, as noted above, a variety of different labels, as are well known to those of skill in the art, including, but not limited to, tetramethylrhodamine-isothiocyanate (TRITC), fluorescein-isothiocyanate (FITC), and succidimidyl ester derivatives, thereof, or any other dye molecule that may be reacted to proteins via amino acid side chains such as amine side chains (lysine), thiol side chains (cysteine) or other suitable functional group.

### B. Binding Assay and Chip Readout

Labeled protein samples are incubated with the proteomic microarray chip for periods of time, and under a variety of conditions of pH, salt content and temperature anticipated to modulate the affinity of various proteins to the elements of the array. Generally, the samples are contacted with the microarray by introduction of an appropriate volume of the fluid sample onto the array surface, where introduction can be flooding the surface with the sample, deposition of the sample onto the surface, e.g., with a pipette, immersion of the entire array in the sample, and the like. In many embodiments, the solution is deposited onto the surface and then sandwiched beneath a cover slip or in a sealed chamber.

For example, a 25 µL - 100 µL (typically 50 µL) aliquot of each probe solution may be applied to the surface of a typical microscope slide-sized chip, and a cleaned coverslip placed on top, forming a sandwich of the probe solution on the chip surface. The protein solutions may then be co-incubated with the chip for at least 1 hour, or overnight. After incubation, the coverslip is removed and the chip is washed, for example, in 1X PBS / 0.05% Tween or other suitable buffer containing surfactant. The chip may be washed using a variety of conditions that decrease or increase stringency. These conditions can again be customized to allow, for example, retention of only the most strongly bound proteins. Or, as the case may warrant, less stringent washing may be used to allow visualization of comparatively weaker bound proteins. The choice is likely to be determined by the complexity and diversity of the peptidomimetic (e.g., peptoid) array that is displayed on the chip and the nature of the protein mixture. The washed chips are then dried, for example, under a stream of Argon or Nitrogen.

After suitable washing, the chip is read in an array scanner, such as are well known in the art. The ratio of Cy 3 to Cy 5 for each spot is determined using commercially available software. Spots that show a ratio considerably greater than or less than one are observed, and deemed to be "differential".

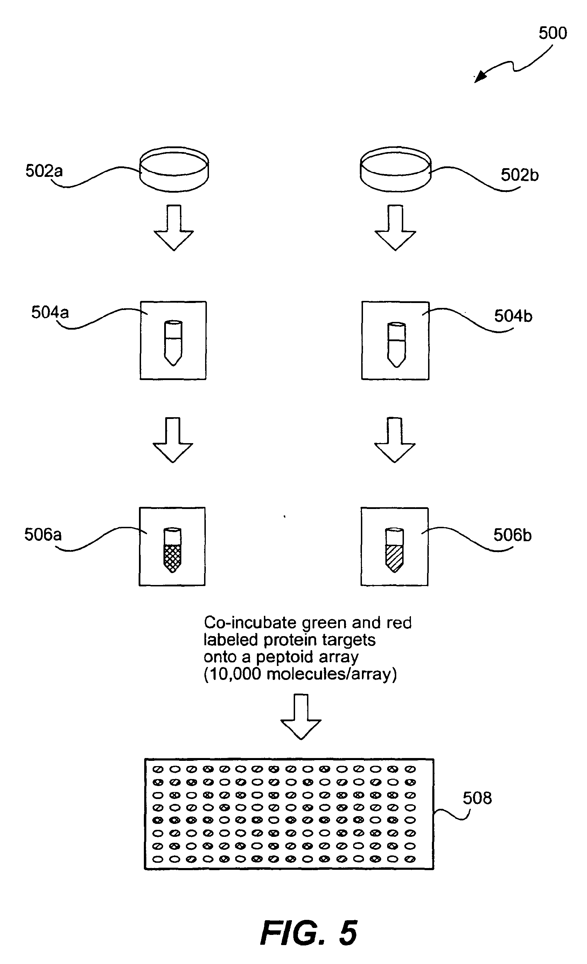

Fig. 5 briefly illustrates a process for conducting a differential proteomic binding assay using protein-binding agent arrays for one embodiment of the invention in accordance with the procedures described above. In Fig. 5, the process (500) begins with the procurement of two biological samples to compare, e.g., an "untreated" cell line 502a and a "treated" cell line 502b. Cell lysates 504a,b are isolated from the cell line samples. The lysates are labeled, for example, the "untreated" cell lysate 504a is labeled with a fluorescent green dye while the "treated" cell lysate 504b is labeled with a fluorescent red dye. The labeled samples 506a,b are then co-incubated on a protein-binding array chip 508 in accordance with the present invention, e.g., an array of peptoids. The protein in the samples can either be denatured or native. For example with the addition of 1-2% SDS the proteins in the samples may be denatured and clusters or hydrophobic interactions minimized or eliminated. Alternatively, the clusters, which may be important in elucidating protein-protein binding pathways, and proteins may be kept in their native states and the results studied. The chip is then read in a array scanner.

### C. Library Sequencing

The sequence of the peptidomimetic that binds a differentially expressed protein may be determined by library sequencing techniques. This can be achieved, for example, by MS/MS methods that allow fragmentation of the peptoid along the amide backbone. These methods are described in PCT Publication No. WO 00/72004. From the mass of the fragmentation product, the structure and sequence of the isolated peptoid can be determined.

### D. Post-Array Processing: Protein Isolation, Purification and Identification

Once a protein or set of proteins is determined to be differential between samples A and B, it can be isolated by preparing chromatographic supports composed of the same peptidomimetic (e.g., peptoid) identified on the chip.

In accordance with one embodiment of that disclosure, a peptoid sequence that produces a differential on the chip may be synthesized on hydrophilic resins that are hydrophobically "masked" during peptoid synthesis. After synthesis, the resin in "unmasked" to reveal hydrophilic groups compatible with biological solutions. Such a resin is immediately available for protein binding / isolation experiments.

Once the protein is isolated, it's sequence can be determined using standard techniques such as MALDI / TOF mass spectrometry or trypsin digests.

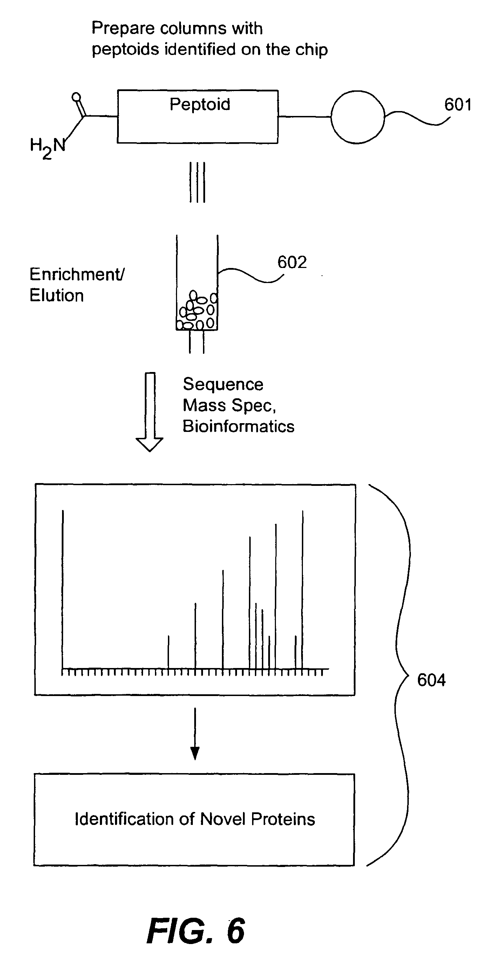

Fig. 6 briefly illustrates aspects of post-array processing in accordance with the procedures described above and below. In Fig. 6, the process (600) begins with the preparation of chromatographic separation columns 602 using peptidomimetic agents 601, e.g., peptoids, identified as of interest in a proteomic differential binding assay conducted using a proteomic microarray in accordance with the present invention. An aliquot of the complex sample originally run on the microarray is then run through the column. The protein of interest preferentially binds to the column and is thereby separated form other components of the sample. The bound protein is eluted and may then be used in further analyses 604, such as protein sequencing, tertiary structure determination, etc. In addition, data relating to the identification of the protein may be entered into bioinformatics databases for further research.

Alternatively, the same peptoid that bound the differentially expressed protein could be spotted repetitively on a chip and incubated with the an aliquot of the same lysate. The same protein should bind to the array, but in a much larger area than just the one spot on the original chip. Laser desorption mass spectrometry can then be used to sequence the protein directly from the chip.

### E. Other Applications

The anticipated uses of proteomic microarray chips in accordance with the present invention are broad. In general, the applications have in common the identification of a protein or set of proteins that are over-expressed or under-expressed in one complex mixture relative to another (or present/absent, such as in the case of a diagnostic protein). Those skilled in the art will recognize that embodiments of the present invention are compatible with a wide variety of assay formats including sandwich assay, such as ELISA.

As described above, proteomic microarrays may be used to determine differential expression of proteins in complex solutions by alternatively labeling (e.g., Cy 3 for one sample and Cy 5 for another) the two or more protein solutions to be compared. The chips may be used to find novel protein targets for later high throughput screening assays. In another particularly powerful application, the methodology may be used to purify a recombinant protein that is overexpressed in a particular host such as yeast or baculovirus. The sample that contains the expressed protein is compared to the sample that does not by co-binding the alternatively labeled samples on the chip, and looking for differentials. The procedure identifies peptoids on the array that bind with reasonable affinity and specificity to the expressed protein. These same peptoids are then used for generating chromatographic resins for the isolation and purification of the recombinant protein of interest.

In an analogous manner, the proteomic microarry chips may be used to find protein markers in plasma or serum that may be diagnostic of particular disease states such as cancer, HIV, or diabetes, or to find novel targets for drug screening. Also, once a set of ligands has been identified for particular groups of proteins, it is possible to monitor the expression levels of these proteins to decipher mechanisms of drug action. In that regard, the new ligands may be used as probes of protein abundance, analogous to the ways in which antibodies are currently used to determine protein abundance. In addition, by examining the proteins from virulent and non-virulent strains of bacteria or viruses, one can determine unique virulence factors that result in infectious disease. Once these virulence factors are identified, these proteins can be used as targets for screening new anti-bacterial or anti-viral drugs. Alternatively, the chips provided by the present invention may also be used to discover a variety of peptide, antigen, or protein mimetics. For example, novel mimetic cellular adhesion molecules, mimetic drug candidates, or protein mimetics that may be used as chromatographic supports. In addition, the material spotted on the chip could itself be potential drug candidates or function as an initial scaffold for designing new drug candidates. In such embodiments, high throughput assays to identify potential therapeutic agents can be done directly on the chip.

In one embodiment, the proteomic microarrays of the invention are run in parallel with DNA arrays, and the differential binding results derived from each are compared to identify correlations in gene activity and protein expression. For example, differential binding assays are conducted for complex biological samples on both proteomic and DNA arrays. Separate aliquots from the samples are labeled and contacted with a proteomic microarray in accordance with the present invention and a DNA microarray, such as are well known in the art. The differential protein expression evidenced by the binding results on the proteomic array when compared with those for the DNA array may elucidate relationships between protein expression and gene families whose activation is required for that expression.

In another embodiment of the present invention, a "mixed array" chip of the present invention is provided. Peptoid or other peptidomimetic binding elements attached to the chip surface may be mixed with antibody or protein binding elements such that both types of binding elements are present on the same chip. The antibodies can serve as positive controls, or as a means of monitoring the levels of specific proteins in the mixture being analyzed. The peptoids would provide data on differentials in unknown proteins, whereas antibody spots on the chip would provide data on differential levels of known proteins. For example, if a cell is treated with a certain drug, the protein levels might change up or down. If those proteins have known antibodies, it is possible to monitor the change of these with antibody differentials, while at the same time, look for changes in unknown proteins, with the peptoid differentials.

In one example of this technique, antibody solutions are prepared in the same microtiter plates as the peptoid solutions, but in different wells. The peptoids are already functionalized with thiol anchoring groups. The antibodies are reacted with a reducing agent to reduce the disulfide bonds in the antibody hinge and Fc region (for example, as described in Levison, M.E., et al, (1969), Experentia, vol 25, 126-127 and Blauenstein, P., et al, (1995), Eur. J. Nuclear Med., vol 22, 690-698), thus producing a thiol on the antibody. This allows for spotting of both peptoid and antibody in the same spotting session, thereby creating a "mixed" chip. Alternatively, the antibodies may be bound to immobilized Protein A or Protein G on chip surfaces, while peptoids are attached to avidin or streptavidin, presenting a mixed surface for display. Or, antibodies may simply be biotinylated and spotted together with biotinylated peptoids or peptides onto avidin-coated chips.

Another technique that may be combined with the proteomic microarray techniques of the present invention is the MS/MS macromolecular structural analysis technique. In this way, the combination of techniques can be used to identify a protein of interest, enrich and isolate it, sequence the protein, and elucidate aspects of its tertiary protein structure.

Data relating to the identification and post-array processing of proteins of interest may also be entered into bioinfomatics databases. The data may be correlated with other biological data therein for further research.

### 4. Kits

Also provided by the subject invention are kits for performing proteomic binding assays using the subject arrays. Such kits according to the present invention will at least include an array according to the invention. The kits may be configured for analytical or diagnostic purposes, and may further include one or more additional reagents employed in the method for which the array is intended. For example, the kit may include various receptacles, labels, buffer solutions, tools and any other material necessary to conduct a proteomic binding assay. Kits in accordance with the present invention may also be configured to receive samples for analysis and thereafter perform the steps necessary for a binding assay in accordance with the invention without further user manipulation.

### EXAMPLES

The following examples provide details concerning the synthesis and characteristics of the proteomic arrays in accordance with the present invention, their components, and applications. It should be understood the following is representative only, and that the invention is not limited by the detail set forth in these examples.

### Example 1: Preparation of microtiter plates containing one compound per well

Preparation of one (1) 96-well plate containing 96 single compounds (0.8 mM) in a 1:1 DMSO/PBS solution: A siliconized vial (8 ml, 2 dram) was loaded with a one compound/bead library synthesized on Rink polystyrene macro-bead solid support (66 mg, ∼1320 beads, 40 nanomole/bead, 0.75 mmol/g, 425-500 um, Polymer Laboratories) that contains a total of 121 possible compounds. The beads were swollen in dichloroethane (DCE, 4 ml) for 24 hours and sieved over stainless steel mesh (600 um). Using the resulting dichloroethane bead slurry, 96 beads were individually picked and relocated into a polypropylene 96-well plate (200 µL, conical bottom) in order to obtain one bead per well over 96 wells. The resulting 96-well plate, (the 'grandmother' plate), was transferred to a speed-vac evaporator Savant AES200 equipped with a 96-well plate carrier rotor and the remaining DCE was removed from the plate. A cleavage cocktail (TFA/TES/H2O/DCE, 46:2:2:50, 75 µL) was then added to each bead containing well in the plate. After an hour the plate was transferred to the speed-vac evaporator to remove most of the volatiles from the plate. Each well was treated with acetonitrile (CH₃CN, 10 µL) and agitated for 10 min using a microtiter plate shaker. The plate was transferred to a speed-vac evaporator and the remaining volatiles were removed from the plate for 10 minutes. Every single compound in each well was dissolved in dimethylsulfoxide (DMSO, 25 µL) and relocated in a 96-well filter plate made of polystyrene, equipped with a poly-propylene membrane (0.45 µm). The 96-well filter plate was stacked over a polypropylene 96-well plate (200 µL, conical bottom) and the assembly was transferred to a Beckman GS-6R centrifuge programmed for 5 minutes at 3000 RPM (∼2000G) at 15 °.

This procedure provided the 'mother' plate composed of filtered 96 DMSO solutions (1.6 mM) of one compound in 96 wells. To form the 'daughter plate' (used for spotting), an aliquot of every DMSO solution (5 µL) was transferred to a polypropylene 96-well plate (200 µL, conical bottom). Then, every well in the daughter plate was mixed with degassed solution PBS (5 µL) and stored at -20°C. Alternatively, each compound in each well may be dissolved in 20 µL of 50% DMSO / water. In the case of a plate with a conical bottom, the plate may be centrifuged at 1,800 rpm for 5 minutes to immobilize the bead at the bottom of the conical well, followed by relocation of 10 uL of the clear supernatant into a daughter plate that is ready for spotting.

The same can be done for 384 beads in 384 well plates, or 1,024 beads in 1,024-well plates, etc. For spotting, any suitable spotting device can be used, such as a Molecular Dynamics Generation II (for 96-well) or Generation III (384-well) spotters.

### Example 2: Functionalization of solid supports for spotting

Gold or aluminum reflective microscope slides were used as substrates for spotting a library of peptoids. In one example, the peptoid is functionalized with a thiol endgroup and the surface is functionalized with a maleimide. Upon spotting, the thiol on the peptoid reacts with the maleimide on the surface to form a covalent thioether attachment. Gold surfaces activated with maleimide were prepared as follows: 1) Gold-coated microscope slides (1200 Angstroms Au, 30-50 Angstroms Ti or Cr) were cleaned with Chromic acid cleaning solution for 15 minutes and rinsed with HPLC grade water. 2) Gold slides were dipped into 1-5 mM amino-modified thiol (1-Mereaptoundecyldiethoxyamine; a C-11 alkyl, two ethylene oxides, and an amine; alternatively, C-2 to C-20 alkyl groups and/or ethoxy or triethoxy groups could be used in such a compound) for 1-24 hours at room temperature or at 45 or 60°C. The slides were rinsed four times in absolute ethanol and dried under a steam of Nitrogen. 3) The amino-modified gold slides were dipped into a solution (50-100 uM) of succinimidyl 4-(N-maleimidomethyl)-cyclohexane-1-carboxylate (SMCC) to render a surface that presents the maleimide functional groups.

Aluminum slides were made or purchased from Amersham-Pharmacia, coated with a 1000 - 1400 Angstrom layer of silicon oxide, and a layer of aminopropysilane (APS). The amino-modified Al surfaces are functionalized with SMCC as described above.

In some cases, after spotting the peptoid library, the aluminum slides were etched in a solution of 0.1% SDS / 5X SSC at 60 degrees C for 2 hours. The etched slides exhibit a reduced oxide layer thickness (200 - 900 Angstroms) to allow amplification of both the Cy5 and Cy3 signals.

### Example 3: Derivatization of Aluminum / SiO₂ surfaces with avidin

Aluminum slides coated with aminosilane were dipped into a solution of NHS-LC-LC-biotin ("LC" refers to 6-aminohexanoyl and "NHS" refers to N-hydroxysuccinimidyl) (commercially available from Pierce), depicted in Fig. 7A, that was 0.39mM in PBS buffer. The slides were coated for 1.5 hours with shaking at 80 rpm. After attachment of biotin, the slides were rinsed with water, then dipped in a solution of lug/ml - lmg/ml avidin, streptavidin or neutravidin in PBS buffer for 2 hours, stirring at 70rpm. The slides were rinsed with water and ready for spotting biotinylated peptides or peptoids. Fig. 7B depicts a representation of an avidin-derviatized aluminum slide spotted with a biotinylated protein-binding agent in accordance with one embodiment of the present invention.

The various surface modification steps was followed using ellipsometry to note the thickness changes. A thickness change increase of 40-45 angstroms was reproducibly recorded after the addition of avidin to the surface layers.

### Example 4: Protein labeling

Protein solutions were adjusted to a concentration of 1 mg/mL in 0.1 M sodium carbonate, pH 9.3 and a volume of 0.1-1 mL, and mixed with bifunctional or mono-functional amine-reactive cyanine dye (Cy3 or Cy5, Amersham Pharmacia). The protein was purified from the unreacted dye by size exclusion chromatography using a Sephadex G-25 packing in a 5 cm long, 1.7 cm diameter column with a 1 mL load, 0.5 mL fractions, and a dilution factor of 3.5.

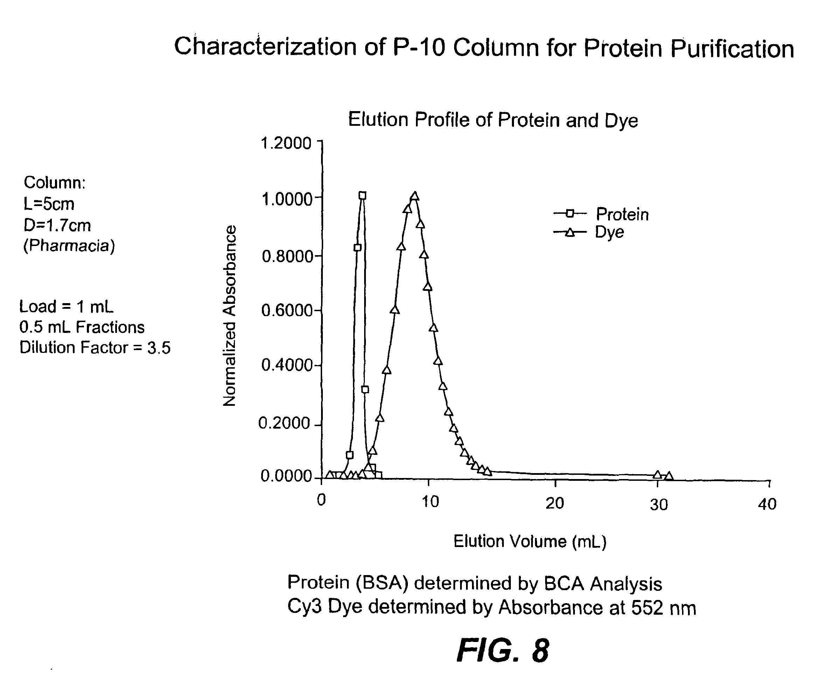

Fig. 8 provides a graph of the results illustrating that size exclusion chromatography of the components efficiently separates the labeled protein from the unreacted dye. The graph shows the different elution profiles for the protein (BSA standard) and dye molecules.

### Example 5: Chip binding experiments

In some instances, the chips spotted with the peptoid library are chemically blocked with cysteine, mercaptoethanol or other suitable hydrophilic thiol. The chips are blocked with protein such as 2% BSA / PBS, 10% non-fat dry milk or 1% casein for at least 1 hour, rinsed with water and dried. The labeled protein probe solution is diluted accordingly (typically to 20 - 1000 ng total protein) with the blocking solution (for the etched A1 slides, detection limits of a few picograms have been observed). A 30 - 100 µL aliquot of the probe solution is applied to the chip surface, and a clean coverslip placed on top, forming a sandwich of the probe solution on the chip surface. The protein solution is incubated with the chip for at least 1 hour. The coverslip is removed in 1X PBS / 0.05% Tween or other suitable buffer containing surfactant. The chip is then washed in 1X PBS / 0.05% Tween or other suitable buffer / surfactant system. The chips are further rinsed with water, dried under a stream of Argon or Nitrogen and scanned.

### Example 6: Sample Chip

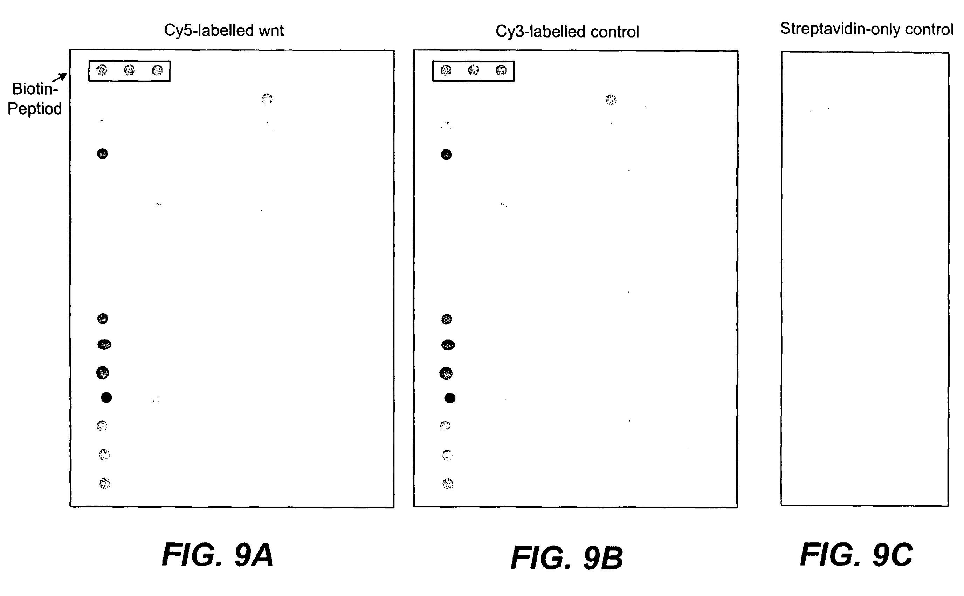

A microarray chip bearing a library of 1,000 peptoid-based protein-binding agents was prepared in accordance with the present invention. Streptavidin was spiked into cells induced to express proteins in the wnt pathway and into control cell lysate and the entire mixture labeled with Cy3 (control) or Cy5 (wnt). The mixture was then incubated with the chip (6.3 ng total protein/chip; 31 pg streptavidin/chip), which had a biotin-displaying peptoid in it's upper left corner. Figs. 9A and 9B show 1/6 of the incubated chip. As shown, in addition to the biotin peptoid spots, many other peptoid spots are binding to the protein in the lysate. By comparing the relative signals from the Cy5 and Cy3 channels, differentials can be identified. Fig. 9C shows ½ of a streptavidin-only control chip (31 pg streptavindin/chip). Biotin peptoid spots are visible in the upper left corner.

### Example 7: Demonstration Chip

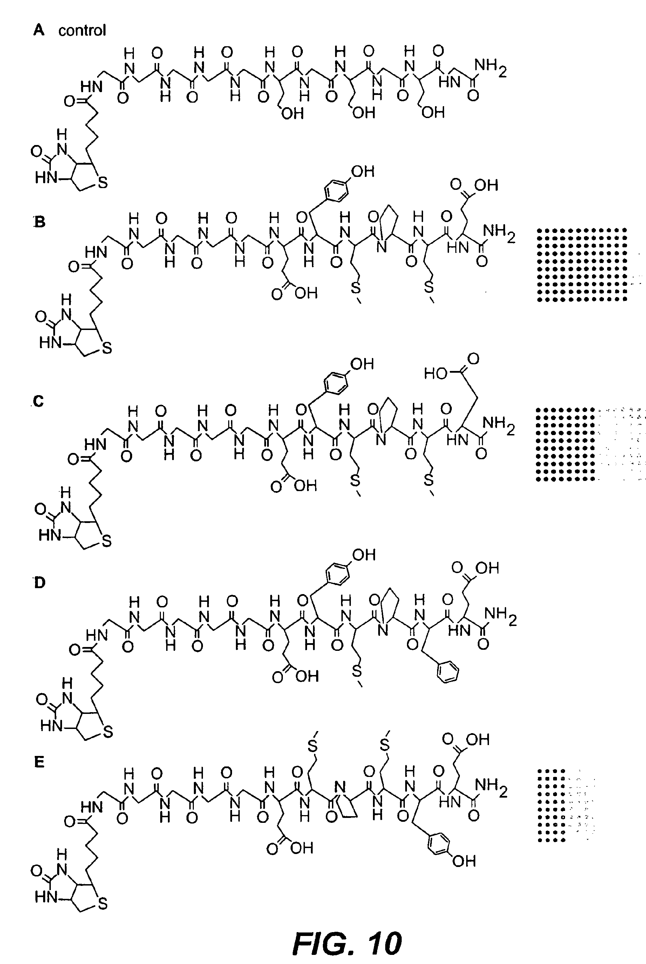

As illustrated in Fig. 10, hexameric peptides of different and known affinity to the "anti-glu" antibody were synthesized with a biotin anchoring group and heptameric gycyl linker. To prove the concept of the present invention, the biotinylated peptides were spotted onto avidin-treated slides as described in the present disclosure. The slides were blocked with casein and probed with Cy5-labelled antiglu antibodies. The gradations in signal intensity correlate with the known differences (e.g., measured by direct ELISA) in affinity between the peptides and their cognate antibody probe.

### Example 8: Comparison of Surface Attachment Systems

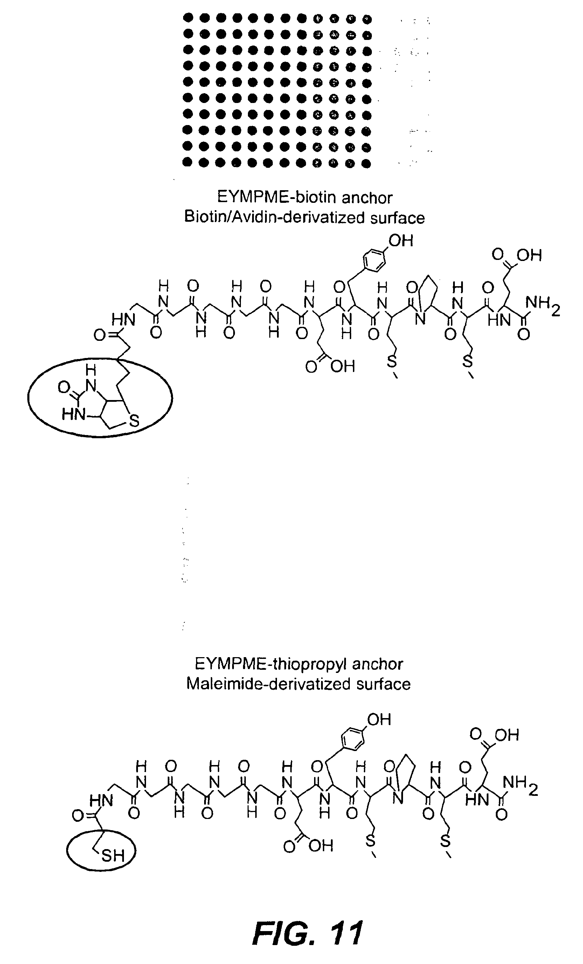

Fig. 11 depicts the dependence of signal strength on the mode of surface attachment of a protein-binding agent to a substrate. In this example, signal increases of 1000X are observed for biotin / avidin immobilization compared to thiol / maleimide (signal results depicted above surface attachment description and molecular structure).

### Example 9: Preparation of Protein-Coated Slide Surface for Antibody Display

To prepare Protein A- or Protein G-modified side surfaces, a slide coated with avidin (prepared as described above) was immersed in a solution of biotinylated Protein A or Protein G (0.5 - 1mg/mL in PBS buffer, purchased from Pierce, product numbers 29989zz and 29988zz) for 2 hours at room temperature. The slides were then rinsed with de-ionized distilled water and blown dry with Nitrogen or Argon.

### Conclusion

Although the foregoing invention has been described in some detail for purposes of clarity of understanding, it will be apparent that certain changes and modifications may be practiced within the scope of the invention. It should also be noted that there are many alternative ways of implementing both the process and apparatus of the present invention. Accordingly, the present embodiments are to be considered as illustrative and not restrictive, and the invention is not to be limited to the details given herein.

## Claims

1. An array of protein-binding agents stably attached to the surface of a solid support, said array comprising:
a solid substrate having a substantially planar aluminium surface, the aluminium coated with a silicon dioxide coating, wherein the silicon dioxide coating has a thickness of about 20 nm to about 90 nm and is capable of enhancing amplification of a fluorescent signal;
a plurality of different protein-binding agents bound to said substrate, each of said protein-binding agents comprising,
an anchoring segment stably bound to the substrate surface,
a peptidomimetic protein-binding segment,
and
a linker segment connecting and separating the anchoring and peptidomimetic segments.

2. The array of claim 1, wherein said substrate is one of glass, plastic or metal.

3. The array of claim 1, wherein said peptidomimetic segment is a peptoid.

4. The array of claim 1, wherein said linker segment is selected from the group consisting of C2-C100 aliphatic chains, polyethylene oxide, and orthogonal peptidomimetic or peptide oligomers.

5. The array of claim 1, wherein said anchoring segment is a thiol.

6. The array of claim 1, wherein said anchoring segment is biotin.

7. The array of claim 1, wherein said metal substrate surface is further coated with a functionalized one of an amino-modified thiol and a siloxane beneath said anchoring segment.

8. The array of claim 7, wherein said aminothiol or amino-silane is functionalized with a maleimide.

9. The array of claim 8, wherein said anchoring segment is a thiol.

10. The array of claim 7, wherein said aminothiol or aminosilane is functionalized with one of a hydrazide, aminooxy, N-hydroxysuccinimide, anhydride, aldehyde, disulfide, thiol, azide and phosphine.

11. The array of claim 7, wherein said metal substrate surface is further coated with an avidin protein beneath said anchoring segment.

12. The array of claim 11, wherein said avidin protein is selected from the group consisting of avidin, streptavidin, neutravidin and analogs.

13. The array of claim 11, wherein said anchoring group is biotin.

14. The array of claim 11, wherein said avidin protein is attached to the metal substrate surface via an NHS-LC-LC-biotin moiety.

15. An array of protein-binding agents stably associated with the surface of a solid support, said array comprising:
a solid support having a substantially planar aluminum surface, the aluminium coated with a silicon dioxide coating, wherein the silicon dioxide coating has a thickness of about 20 nm to about 90 nm and is capable of enhancing amplification of a fluorescent signal, and wherein the surface is further coated with a maleimide-functionalized aminothiol or amino-silane;
a plurality of different protein-binding agents bound to said substrate, each of said protein-binding agents comprising,
a thiol substrate anchoring segment stably bound to the maleimide-presenting substrate surface,
a peptoid protein-binding segment, and
an aliphatic linker segment connecting and separating the anchoring and peptidomimetic segments.

16. The array of claim 15, wherein said maleimide-functionalized aminothiol or aminosilane comprises a spacer.

17. An array of protein-binding agents stably associated with the surface of a solid support, said array comprising:
a solid support having a substantially planar aluminum surface, the aluminium coated with a silicon dioxide coating, wherein the silicon dioxide coating has a thickness of about 20 nm to about 90 nm and is capable of enhancing amplification of a fluorescent signal; and wherein the surface is further coated with an avidin-functionalized aminosilane or aminothiol;
a plurality of different protein-binding agents bound to said substrate, each of said protein-binding agents comprising,
a biotin substrate anchoring segment stably bound to the avidin-presenting substrate surface,
a peptoid protein-binding segment, and
an orthogonal peptide linker segment connecting and separating the anchoring and peptidomimetic segments.

18. The array of claim 15, wherein said avidin-functionalized aminosilane or aminothiol comprises an NHS-LC-LC-biotin moiety.

19. A method of making an array comprising a plurality of different protein-binding agents stably associated with the surface of a solid support, said method comprising:
preparing for bonding a solid substrate having a substantially planar aluminum surface, the aluminium coated with a silicon dioxide coating, wherein the silicon dioxide coating has a thickness of about 20 nm to about 90 nm and is capable of enhancing amplification of a fluorescent signal;
contacting a plurality of different protein-binding agents with said substrate under conditions sufficient for said protein-binding agents to become bound to said substrate surface, each of said protein-binding agents comprising,
a substrate anchoring segment,
a peptidomimetic protein-binding segment, and
a linker segment connecting and separating the anchoring and peptidomimetic segments;
whereby said array is produced.

20. The method of claim 19, wherein said contacting comprises spotting a droplet of a solution of each of said protein-binding agents in a different location on said substrate surface under conditions such that binding of the protein-binding agents to the substrate surface is complete before the droplet evaporates.

21. The method of claim 20, wherein said substrate surface comprises a gold coating and preparing for bonding comprises cleaning said gold coating.

22. The method of claim 21, wherein said anchoring segment is a thiol.

23. The method of claim 20, wherein said substrate surface comprises a metal coating selected from gold and aluminum, and preparing for bonding comprises cleaning and coating said metal coating with a functionalized aminothiol or aminosilane.

24. The method of claim 23, wherein said aminothiol or aminosilane is aminopropylsilane.

25. The method of claim 24, wherein said aminopropylsilane is functionalized with a maleimide.

26. The method of claim 25, wherein said substrate anchoring segment is a thiol.

27. The method of claim 23, wherein said anchoring segment is biotin.

28. The method of claim 23, wherein said aminothiol or amino-silane is functionalized with one of a hydrazide, aminooxy, N-hydroxysuccinimide, anhydride, aldehyde, disulfide, thiol, azide and phosphine.

29. The method of claim 23, wherein said metal substrate surface is further coated with an avidin protein beneath said anchoring segment.

30. The method of claim 29, wherein said avidin protein is selected from the group consisting of avidin, streptavidin, neutravidin and analogs.

31. The method of claim 29, wherein said anchoring group is biotin.

32. The method of claim 29, wherein said avidin protein is attached to the metal. substrate surface via an NHS-LC-LC-biotin moiety.

33. The method of claim 19, wherein said peptidomimetic segment is a peptoid.

34. The method of claim 19, wherein said linker segment is selected from the group consisting of C2-C100 aliphatic chains, polyethylene oxide, and orthogonal peptidomimetic or peptide oligomers.

35. A method of making an array comprising a plurality of different protein-binding agents stably associated with the surface of a solid support, said method comprising:
generating a library of protein-binding agents, comprising,
a substrate anchoring segment,
a peptidomimetic segment, and
a linker segment connecting and separating the anchoring and peptidomimetic segments;
distributing protein-binding agents from said library into individual storage receptacles for each different protein-binding agent;
preparing a plurality of said different protein-binding agents for binding to a solid substrate;
preparing a solid substrate having a substantially planar aluminum surface for binding with a plurality of said different protein-binding agents, the aluminium coated with a silicon dioxide coating, wherein the silicon dioxide coating has a thickness of about 20 nm to about 90 nm and is capable of enhancing amplification of a fluorescent signal;
whereby said array is produced.

36. The method of claim 35, wherein said substrate surface comprises a metal coating selected from gold and aluminum, and preparing for bonding comprises cleaning and coating said metal coating with a functionalized aminothiol or aminosilane.

37. The method of claim 36, wherein said aminothiol or aminosilane is functionalized with a maleimide.

38. The method of claim 37, wherein said substrate anchoring segment is a thiol.

39. The method of claim 36, wherein said metal substrate surface is further coated with an avidin protein beneath said anchoring segment.

40. The method of claim 39, wherein said anchoring group is biotin.

41. A method of performing a differential binding assay, comprising:
fluorescently labeling proteins in a protein-containing biological sample solution;
contacting an aliquot of said labeled protein-containing biological sample solution with an array according to claim 1;
analyzing the array to determine differential binding of proteins in the sample to protein-binding agents of the array.

42. The method of claim 41, wherein said peptidomimetic segment is a peptoid.

43. The method of claim 42, wherein each of said different protein-binding agents corresponds to a different source receptacle containing that agent.

44. The method of claim 43, further comprising selecting a protein-binding agent of interest based on the differential binding assay results.

45. The method of claim 44, further comprising sequencing the peptidomimetic segment of the selected protein-binding agent.

46. The method of claim 44, further comprising subjecting the peptidomimetic segment of the selected protein-binding agent to structural analysis by mass spectroscopy.

47. The method of claim 44, further comprising enriching the protein-containing biological sample solution with a protein which preferentially binds to the selected protein-binding agent by applying a second aliquot of the protein-containing biological sample solution to a separation column, said separation column comprising a chromatography support displaying the selected protein-binding agent.

48. The method of claim 47, further comprising sequencing said enriched protein.

49. The method of claim 47, further comprising subjecting the enriched protein to structural analysis by mass spectroscopy.

50. The method of claim 41, further comprising contacting an aliquot of said biological sample solution containing labeled cDNA or messenger RNA with a DNA array, analyzing the DNA array to determine differential binding of nucleic acids in the sample to elements of the DNA array, and comparing the differential binding results of the two arrays to identify correlations in gene activity and protein expression.

51. The method of claim 41, wherein the fluorescent labels are amine reactive dyes.

52. The method of claim 51, wherein the fluorescent label is one or more of Cyanine 3 and Cyanine 5.

53. A kit for use in performing a differential binding assay according to claim 41, said kit including an array according to claim 1.

54. The kit of claim 53, further comprising one or more reagents for conducting a differential binding assay comprising a plurality of fluorescent labels for proteins.

55. The kit of claim 54, wherein the fluorescent labels are amine reactive dyes.

56. The kit of claim 55, wherein the amine reactive dyes are Cyanine 3 and Cyanine 5.

57. A mixed array of protein-binding agents stably attached to the surface of a solid support, said array comprising:
a solid substrate having a substantially planar aluminium surface coated with a silicon dioxide coating, wherein the silicon dioxide coating has a thickness of about 20 nm to about 90 nm and is capable of enhancing amplification of a fluorescent signal;
a plurality of different protein-binding agents bound to said substrate, each of said protein-binding agents comprising,
an anchoring segment stably bound to the substrate surface, a peptidomimetic protein-binding segment, and
a linker segment connecting and separating the anchoring and peptidomimetic segments; and
a plurality of different antibodies bound to said substrate.

58. A system for performing a differential binding assay, said system comprising:
an array according to claim 1;
one or more fluorescently labeled proteins bound to one or more of the protein binding agents; and
a fluorescent signal array scanner.

59. The system of claim 58, wherein the fluorescent label is one or more of Cyanine 3 and Cyanine 5.

## Patentansprüche

1. Array aus Protein-Bindungsmitteln, die stabil an die Oberfläche eines festen Trägers gebunden sind, wobei der Array folgendes umfasst:
ein festes Substrat mit einer im Wesentlichen planaren Aluminiumoberfläche, wobei das Aluminium mit einer Silikondioxidbeschichtung beschichtet ist, wobei die Silikondioxidbeschichtung eine Dicke von etwa 20 nm bis etwa 90 nm aufweist und in der Lage ist, die Amplifikation eines Fluoreszenzsignals zu verstärken;
eine Mehrzahl von verschiedenen Protein-Bindungsmitteln, die an das Substrat gebunden sind, wobei jedes der Protein-Bindungsmittel folgendes umfasst:
ein Verankerungssegment, das stabil an die Oberfläche des Substrats gebunden ist,
ein peptidomimetisches, proteinbindendes Segment, und
ein Verbindungssegment, welches das Verankerungssegment und das peptidomimetische Segment verbindet und trennt.

2. Array nach Anspruch 1, bei dem das Substrat entweder aus Glas, Plastik oder Metall ist.

3. Array nach Anspruch 1, bei dem das peptidomimetische Segment ein Peptoid ist.

4. Array nach Anspruch 1, bei dem das Verbindungssegment ausgewählt ist aus der Gruppe bestehend aus C2-C100 aliphatischen Ketten, Polyethylenoxid, und orthogonalen peptidomimetischen Oligomeren oder Peptid-Oligomeren.

5. Array nach Anspruch 1, bei dem das Verankerungssegment ein Thiol ist.

6. Array nach Anspruch 1, bei dem das Verankerungssegment Biotin ist.

7. Array nach Anspruch 1, bei dem die Oberfläche des Metallsubstrats ferner unter dem Verankerungssegment entweder mit einem funktionalisierten, aminomofifizierten Thiol oder mit einem funktionalisierten Siloxan beschichtet ist.

8. Array nach Anspruch 7, bei dem das Aminothiol oder das Aminosilan mit einem Maleimid funktionalisiert ist.

9. Array nach Anspruch 8, bei dem das Verankerungssegment ein Thiol ist.

10. Array nach Anspruch 7, bei dem das Aminothiol oder das Aminosilan mit Hydrazid, Aminooxy, N-Hydroxysuccinimid, Anhydrid, Aldehyd, Disulfid, Thiol, Azid oder Phosphin funktionalisiert ist.

11. Array nach Anspruch 7, bei dem die Oberfläche des Metallsubstrats ferner unter dem Verankerungssegment mit einem Avidinprotein beschichtet ist.

12. Array nach Anspruch 11, bei dem das Avidinprotein ausgewählt ist aus der Gruppe bestehend aus Avidin, Streptavidin, Neutravidin und Analoga.

13. Array nach Anspruch 11, bei dem die Verankerungsgruppe Biotin ist.

14. Array nach Anspruch 11, bei dem das Avidinprotein über eine NHS-LC-LC-Biotin-Gruppe an die Oberfläche des Metallsubstrats gebunden ist.

15. Array aus Protein-Bindungsmitteln, die stabil mit der Oberfläche eines festen Trägers assoziiert sind, wobei der Array folgendes umfasst:
einen festen Träger mit einer im Wesentlichen planaren Aluminiumoberfläche, wobei das Aluminium mit einer Silikondioxidbeschichtung beschichtet ist, wobei die Silikondioxidbeschichtung eine Dicke von etwa 20 nm bis etwa 90 nm aufweist und in der Lage ist, die Amplifikation eines Fluoreszenzsignals zu verstärken, und wobei die Oberfläche ferner mit einem Maleimidfunktionalisierten Aminothiol oder Aminosilan beschichtet ist;
eine Mehrzahl von verschiedenen Protein-Bindungsmitteln, die an das Substrat gebunden sind, wobei jedes dieser Protein-Bindungsmittel folgendes umfasst:
ein Thiol-Substratverankerungssegment, das stabil an die Maleimid-präsentierende Oberfläche des Substrats gebunden ist,
ein Peptoid-Protein-Bindungssegment; und
ein aliphatisches Verbindungssegment, welches das Verankerungssegment und das peptidomimetische Segment verbindet und trennt.

16. Array nach Anspruch 15, bei dem das Maleimidfunktionalisierte Aminothiol oder Aminosilan einen Abstandshalter umfasst.

17. Array aus Protein-Bindungsmitteln, die stabil mit der Oberfläche eines festen Trägers assoziiert sind, wobei der Array folgendes umfasst:
einen festen Träger mit einer im Wesentlichen planaren Aluminiumoberfläche, wobei das Aluminium mit einer Silikondioxidbeschichtung beschichtet ist, wobei die Silikondioxidbeschichtung eine Dicke von etwa 20 nm bis etwa 90 nm aufweist und in der Lage ist, die Amplifikation eines Fluoreszenzsignals zu verstärken, und wobei die Oberfläche ferner mit einem Avidinfunktionalisierten Aminosilan oder Aminothiol beschichtet ist;
eine Mehrzahl von verschiedenen Protein-Bindungsmitteln, die an das Substrat gebunden sind, wobei jedes der Protein-Bindungsmittel folgendes umfasst:
ein Biotin-Substratverankerungssegment, das stabil an die Maleimid-präsentierende Oberfläche des Substrats gebunden ist,
ein Peptoid-Protein-Bindungssegment, und
ein Verbindungssegment aus einem orthogonalen Peptid, welches das Verankerungssegment und das peptidomimetische Segment verbindet und trennt.

18. Array nach Anspruch 15, bei dem das Avidin-funktionalisierte Aminosilan oder Aminothiol eine NHS-LC-LC-Biotin-Gruppe umfasst.

19. Verfahren zur Herstellung eines Arrays, der eine Mehrzahl von verschiedenen Protein-Bindungsmitteln umfasst, die stabil mit der Oberfläche eines festen Trägers assoziiert sind, wobei das Verfahren Schritte umfasst, bei denen man:
ein festes Substrat mit einer im Wesentlichen planaren Aluminiumoberfläche für die Bindung vorbereitet, wobei das Aluminium mit einer Silikondioxidbeschichtung beschichtet ist, wobei die Silikondioxidbeschichtung eine Dicke von etwa 20 nm bis etwa 90 nm aufweist und in der Lage ist, die Amplifikation eines Fluoreszenzsignals zu verstärken;
eine Mehrzahl von verschiedenen Protein-Bindungsmitteln mit dem Substrat unter Bedingungen in Kontakt bringt, die ausreichen, um die Protein-Bindungsmitteln an die Oberfläche des Substrats zu binden, wobei jedes Protein-Bindungsmittel folgendes umfasst:
ein Substratverankerungssegment,
ein peptidomimetisches Protein-Bindungssegment, und
ein Verbindungssegment, welches das Verankerungssegment und das peptidomimetische Segment verbindet und trennt;
wodurch der Array hergestellt wird.

20. Verfahren nach Anspruch 19, bei dem das Inkontaktbringen das Platzieren eines Tropfens einer Lösung von jedem der Protein-Bindungsmittel an eine unterschiedliche Stelle der Substratoberfläche umfasst, unter Bedingungen, die dazu führen, dass die Bindung der Protein-Bindungsmittel an die Substratoberfläche abgeschlossen ist, bevor der Tropfen verdampft.

21. Verfahren nach Anspruch 20, bei dem die Substratoberfläche eine Goldbeschichtung umfasst, und das Vorbereiten für die Bindung die Reinigung der Goldbeschichtung umfasst.

22. Verfahren nach Anspruch 21, bei dem das Verankerungssegment ein Thiol ist.

23. Verfahren nach Anspruch 20, bei dem die Oberfläche des Substrats eine Metallbeschichtung umfasst, die ausgewählt ist aus Gold und Aluminium, und bei dem das Vorbereiten für die Bindung die Reinigung und Beschichtung der Metallbeschichtung mit einem funktionalisierten Aminothiol oder einem funktionalisierten Aminosilan umfasst.

24. Verfahren nach Anspruch 23, bei dem das Aminothiol oder Aminosilan ein Aminopropylsilan ist.

25. Verfahren nach Anspruch 24, bei dem das Aminopropylsilan mit einem Maleimid funktionalisiert ist.

26. Verfahren nach Anspruch 25, bei dem das Substratverankerungssegment ein Thiol ist.

27. Verfahren nach Anspruch 23, bei dem das Verankerungssegment Biotin ist.

28. Verfahren nach Anspruch 23, bei dem das Aminothiol oder Aminosilan mit einem von Hydrazid, Aminooxy, N-Hydroxysuccinimid, Anhydrid, Aldehyd, Disulfid, Thiol, Azid und Phosphin funktionalisiert ist.

29. Verfahren nach Anspruch 23, bei dem die Oberfläche des Metallsubstrats ferner unter dem Verankerungssegment mit einem Avidinprotein beschichtet ist.

30. Verfahren nach Anspruch 29, bei dem das Avidinprotein ausgewählt ist aus der Gruppe bestehend aus Avidin, Streptavidin, Neutravidin und Analoga.

31. Verfahren nach Anspruch 29, bei dem die Verankerungsgruppe Biotin ist.

32. Verfahren nach Anspruch 29, bei dem das Avidinprotein über eine NHS-LC-LC-Biotin-Gruppe an die Oberfläche des Metallsubstrats gebunden ist.

33. Verfahren nach Anspruch 19, bei dem das peptidomimetische Segment ein Peptoid ist.

34. Verfahren nach Anspruch 19, bei dem das Verbindungssegment ausgewählt ist aus der Gruppe bestehend aus C2-C100 aliphatische Ketten, Polyethylenoxid und orthogonalen peptidomimetischen Oligomeren oder Peptid-Oligomeren.

35. Verfahren zur Herstellung eines Arrays, der eine Mehrzahl von verschiedenen Protein-Bindungsmitteln umfasst, die stabil mit der Oberfläche eines festen Trägers assoziiert sind, wobei das Verfahren Schritte umfasst, bei denen man:
eine Bibliothek von Protein-Bindungsmitteln erzeugt, die folgendes umfassen:
ein Substratverankerungssegment,
ein peptidomimetisches Segment, und
ein Verbindungssegment, welches das Verankerungssegment und das peptidomimetische Segment verbindet und trennt;
die Protein-Bindungsmittel der Bibliothek in einzelne Lagerungsbehälter für jedes unterschiedliche Protein-Bindungsmittel verteilt;
eine Mehrzahl dieser verschiedenen Protein-Bindungsmittel für die Bindung an ein festes Substrat vorbereitet;
ein festes Substrat mit einer im Wesentlichen planaren Aluminiumoberfläche für die Bindung mit einer Mehrzahl der verschiedenen Protein-Bindungsmittel vorbereitet, wobei das Aluminium mit einer Silikondioxidbeschichtung beschichtet ist, wobei die Silikondioxidbeschichtung eine Dicke von etwa 20 nm bis etwa 90 nm aufweist und in der Lage ist, die Amplifikation eines Fluoreszenzsignals zu verstärken;
wodurch der Array hergestellt wird.

36. Verfahren nach Anspruch 35, bei dem die Oberfläche des Substrats eine Metallbeschichtung umfasst, die ausgewählt ist aus Gold und Aluminium, und bei dem das Vorbereiten für die Bindung die Reinigung und Beschichtung der Metallbeschichtung mit einem funktionalisierten Aminothiol oder einem funktionalisierten Aminosilan umfasst.

37. Verfahren nach Anspruch 36, bei dem das Aminothiol oder Aminosilan mit einem Maleimid funktionalisiert ist.

38. Verfahren nach Anspruch 37, bei dem das Substratverankerungssegment ein Thiol ist.

39. Verfahren nach Anspruch 36, bei dem die Oberfläche des Metallsubstrats ferner unter dem Verankerungssegment mit einem Avidinprotein beschichtet ist.

40. Verfahren nach Anspruch 39, bei dem die Verankerungsgruppe Biotin ist.

41. Verfahren zur Durchführung eines Assays für differentielle Bindung, umfassend:
fluoreszent markierte Proteine in einer proteinhaltigen biologischen Probenlösung;
das Inkontaktbringen eines Aliquots der markierten proteinhaltigen biologischen Probenlösung mit einem Array nach Anspruch 1;
das Analysieren des Arrays, um die differentielle Bindung von Proteinen in der Probe an Protein-Bindungsmittel des Arrays zu bestimmen.

42. Verfahren nach Anspruch 41, bei dem das peptidomimetische Segment ein Peptoid ist.

43. Verfahren nach Anspruch 42, bei dem jedes der verschiedenen Protein-Bindungsmittel einem unterschiedlichen Quellenbehälter entspricht, der dieses Mittel enthält.

44. Verfahren nach Anspruch 43, das ferner die Auswahl eines Protein-Bindungsmittel von Interesse basierend auf den Ergebnissen des Assays für differentielle Bindung umfasst.

45. Verfahren nach Anspruch 44, das ferner die Sequenzierung des peptidomimetischen Segments des ausgewählten Protein-Bindungsmittel umfasst.

46. Verfahren nach Anspruch 44, das ferner einen Schritt umfasst, bei dem das peptidomimetische Segment des ausgewählten Protein-Bindungsmittels einer Strukturanalyse durch Massenspektroskopie unterzogen wird.

47. Verfahren nach Anspruch 44, das ferner das Anreichern der proteinhaltigen biologischen Probenlösung mit einem Protein umfasst, das vorzugsweise an das ausgewählte Protein-bindungsmittel bindet, indem man ein zweites Aliquot der proteinhaltigen Probenlösung auf eine Trennungssäule aufbringt, wobei die Trennungssäule einen chromatographischen Träger umfasst, der das ausgewählte Proteinbindungsmittel zeigt.

48. Verfahren nach Anspruch 47, das ferner die Sequenzierung des angereicherten Proteins umfasst.

49. Verfahren nach Anspruch 47, das ferner einen Schritt umfasst, bei dem das angereicherte Protein einer Strukturanalyse durch Massenspektroskopie unterzogen wird.

50. Verfahren nach Anspruch 41, das ferner das Inkontaktbringen eines Aliquots der biologischen Probenlösung, die markierte cDNA oder mRNA enthält, mit einem DNA-Array, das Analysieren des DNA-Arrays zur Feststellung der differentiellen Bindung von Nukleinsäuren in der Probe an Elemente des DNA-Arrays und den Vergleich der Ergebnisse der differentiellen Bindung der zwei Arrays zur Identifizierung von Korrelationen der Genaktivität und Proteinexpression umfasst.

51. Verfahren nach Anspruch 41, bei dem die fluoreszenten Markierungen aminreaktive Farbstoffe sind.

52. Verfahren nach Anspruch 51, bei dem die Fluoreszenzmarkierung eines oder mehrere von Cyanin 3 und Cyanin 5 sind.

53. Kit zur Verwendung bei der Durchführung eines Assays für differentielle Bindung nach Anspruch 41, wobei das Kit einen Array nach Anspruch 1 umfasst.

54. Kit nach Anspruch 53, das ferner ein oder mehrere Reagenzien zur Durchführung eines Assays für differentielle Bindung umfasst, die eine Mehrzahl von fluoreszenten Markierungen für Proteine umfassen.

55. Kit nach Anspruch 54, bei dem die fluoreszenten Markierungen aminreaktive Farbstoffe sind.

56. Verfahren nach Anspruch 55, bei dem die aminreaktive Farbstoffe Cyanin 3 und Cyanin 5 sind.

57. Gemischter Array aus Protein-Bindungsmitteln, die stabil an die Oberfläche eines festen Trägers gebunden sind, wobei der Assay folgendes umfasst:
ein festes Substrat mit einer im Wesentlichen planaren Aluminiumoberfläche, die mit einer Silikondioxidbeschichtung beschichtet ist, wobei die Silikondioxidbeschichtung eine Dicke von etwa 20 nm bis etwa 90 nm aufweist und in der Lage ist, die Amplifikation eines Fluoreszenzsignals zu verstärken;
eine Mehrzahl von verschiedenen Protein-Bindungsmitteln, die an das Substrat gebunden sind, wobei jedes der Protein-Bindungsmittel folgendes umfasst:
ein Verankerungsmolekül, das stabil an die Oberfläche des Substrats gebunden ist, ein peptidomimetisches Protein-Bindungssegment, und
ein Verbindungssegment, welches das Verankerungssegment und das peptidomimetische Segment verbindet und trennt; und
eine Mehrzahl von verschiedenen Antikörpern, die an das Substrat gebunden sind.

58. System zur Durchführung eines Assays für differentielle Bindung, wobei das System folgendes umfasst:
einen Array nach Anspruch 1;
ein oder mehrere fluoreszenzmarkierte Proteine, die an einen oder mehrere Proteinbindungsmittel gebunden haben; und
einen Scanner für das Fluoreszenzsignal des Arrays.

59. System nach Anspruch 58, bei dem die Fluoreszenzmarkierung eines oder mehrere von Cyanin 3 und Cyanin 5 sind.

## Revendications

1. Matrice d'agents liant des protéines attachés de façon stable sur la surface d'un support solide, ladite matrice comprenant :
un substrat solide ayant une surface d'aluminium sensiblement plane, l'aluminium étant revêtu d'un revêtement de dioxyde de silicium, dans lequel revêtement de dioxyde de silicium a une épaisseur d'environ 20 nm à environ 90 nm et est capable d'améliorer l'amplification d'un signal fluorescent ;
une pluralité de différents agents liant des protéines liés audit substrat, chacun desdits agents liant des protéines comprenant,
un segment d'ancrage lié de façon stable à la surface du substrat,
un segment liant une protéine peptidomimétique, et
un segment lieur connectant et séparant les segments d'ancrage et peptidomimétique.

2. Matrice de la revendication 1, dans laquelle ledit substrat est l'un parmi du verre, du plastique ou du métal.

3. Matrice de la revendication 1, dans laquelle ledit segment peptidomimétique est un peptoïde.

4. Matrice de la revendication 1, dans laquelle ledit segment lieur est choisi dans l'ensemble consistant en chaînes aliphatiques en C2-C100, poly(oxyde d'éthylène), et oligomères peptidomimétiques ou peptidiques orthogonaux.

5. Matrice de la revendication 1, dans laquelle ledit segment d'ancrage est un groupe thiol.

6. Matrice de la revendication 1, dans laquelle ledit segment d'ancrage est la biotine.

7. Matrice de la revendication 1, dans laquelle ladite surface de substrat métallique est en outre revêtue d'un élément fonctionnalisé choisi parmi un groupe thiol et un groupe siloxane modifié par un groupe amino en dessous dudit segment d'ancrage.

8. Matrice de la revendication 7, dans laquelle ledit aminothiol ou aminosilane est fonctionnalisé avec un maléimide.

9. Matrice de la revendication 8 , dans laquelle ledit segment d'ancrage est un groupe thiol.

10. Matrice de la revendication 7, dans laquelle ledit aminothiol ou aminosilane est fonctionnalisé avec un groupe choisi parmi les groupes hydrazide, aminooxy, N-hydroxysuccinimide, anhydride, aldéhyde, disulfure, thiol, azide et phosphine.

11. Matrice de la revendication 7, dans laquelle ladite surface de substrat métallique est en outre revêtue d'une protéine de type avidine en dessous dudit segment d'ancrage.

12. Matrice de la revendication 11, dans laquelle ladite protéine de type avidine est choisie dans le groupe consistant en avidine, streptavidine, neutravidine et leurs analogues.

13. Matrice de la revendication 11, dans laquelle ledit groupe d'ancrage est la biotine.

14. Matrice de la revendication 11, dans laquelle ladite protéine de type avidine est attachée à la surface de substrat métallique par l'intermédiaire d'une partie NHS-LC-LC-biotine.

15. Matrice d'agents liant des protéines associés de façon stable à la surface d'un support solide, ladite matrice comprenant :
un support solide ayant une surface d'aluminium sensiblement plane, l'aluminium étant revêtu d'un revêtement de dioxyde de silicium, lequel revêtement de dioxyde de silicium a une épaisseur d'environ 20 nm à environ 90 nm et est capable d'améliorer l'amplification d'un signal fluorescent, et dans lequel la surface est en outre revêtue d'un aminothiol ou d'un aminosilane à fonction maléimide ;
une pluralité de différents agents liant des protéines liés audit substrat, chacun desdits agents liant des protéines comprenant,
un segment d'ancrage de substrat de type thiol lié de façon stable à la surface du substrat présentant les groupes maléimide,
un segment liant une protéine peptoïde, et
un segment lieur aliphatique connectant et séparant les segments d'ancrage et peptidomimétique.

16. Matrice de la revendication 15, dans lequel ledit aminothiol ou aminosilane à fonction maléimide comprend un espaceur.

17. Matrice d'agents liant des protéines associés de façon stable à la surface d'un support solide, ladite matrice comprenant :
un support solide ayant une surface d'aluminium sensiblement plane, l'aluminium étant revêtu d'un revêtement de dioxyde de silicium, lequel revêtement de dioxyde de silicium a une épaisseur d'environ 20 nm à environ 90 nm et est capable d'améliorer l'amplification d'un signal fluorescent, et dans lequel la surface est en outre revêtue d'un aminosilane ou d'un aminothiol à fonction avidine ;
une pluralité de différents agents liant des protéines liés audit substrat, chacun desdits agents liant des protéines comprenant,
un segment d'ancrage de substrat de biotine lié de façon stable à la surface du substrat présentant les avidines,
un segment liant une protéine peptoïde, et
un segment lieur peptidique orthogonal connectant et séparant les segments d'ancrage et peptidomimétique.

18. Matrice de la revendication 15, dans lequel ledit aminosilane ou aminothiol à fonction avidine comprend une partie NHS-LC-LC-biotine.

19. Procédé de fabrication d'une matrice comprenant une pluralité de différents agents liant des protéines associés de façon stable à la surface d'un support solide, ledit procédé comprenant :
la préparation pour la liaison d'un substrat solide ayant une surface d'aluminium sensiblement plane, l'aluminium étant revêtu d'un revêtement de dioxyde de silicium, lequel revêtement de dioxyde de silicium a une épaisseur d'environ 20 nm à environ 90 nm et est capable d'améliorer l'amplification d'un signal fluorescent ;
la mise en contact d'une pluralité des différents agents liant des protéines avec ledit substrat dans des conditions suffisantes pour que lesdits agents liant des protéines se lient à ladite surface de substrat, chacun desdits agents liant des protéines comprenant,
un segment d'ancrage de substrat,
un segment liant une protéine peptidomimétique, et
un segment lieur connectant et séparant les segments d'ancrage et peptidomimétique ;
grâce auquel ladite matrice est produite.

20. Procédé de la revendication 19, dans lequel ladite étape de contact comprend le dépôt d'une gouttelette d'une solution de chacun desdits agents liant des protéines en un site différent sur ladite surface de substrat, dans des conditions telles que la liaison des agents liant les protéines à la surface de substrat est achevée avant que la gouttelette ne s'évapore.

21. Procédé de la revendication 20, dans lequel ladite surface de substrat comprend un revêtement d'or et la préparation pour la liaison comprend le nettoyage dudit revêtement d'or.

22. Procédé de la revendication 21, dans lequel ledit segment d'ancrage est un groupe thiol.

23. Procédé de la revendication 20, dans lequel ladite surface de substrat comprend un revêtement métallique choisi parmi l'or et l'aluminium, et la préparation pour la liaison comprend le nettoyage et le revêtement dudit revêtement métallique avec un aminothiol ou un aminosilane fonctionnalisé.

24. Procédé de la revendication 23, dans lequel ledit aminothiol ou aminosilane est un aminopropylsilane.

25. Procédé de la revendication 24, dans lequel ledit aminopropylsilane est fonctionnalisé par un maléimide.

26. Procédé de la revendication 25, dans lequel ledit segment d'ancrage du substrat est un groupe thiol.

27. Procédé de la revendication 23, dans lequel ledit segment d'ancrage est la biotine.

28. Procédé de la revendication 23, dans lequel ledit aminothiol ou aminosilane est fonctionnalisé avec un groupe choisi parmi les groupes hydrazide, aminooxy, N-hydroxysuccinimide, anhydride, aldéhyde, disulfure, thiol, azide et phosphine.

29. Procédé de la revendication 23, dans lequel ladite surface de substrat métallique est en outre revêtue d'une protéine de type avidine en dessous dudit segment d'ancrage.

30. Procédé de la revendication 29, dans lequel ladite protéine de type avidine est choisie dans le groupe consistant en avidine, streptavidine, neutravidine et leurs analogues.

31. Procédé de la revendication 29, dans lequel ledit groupe d'ancrage est la biotine.

32. Procédé de la revendication 29, dans lequel ladite protéine de type avidine est attachée à la surface de substrat métallique par l'intermédiaire d'une partie NHS-LC-LC-biotine.

33. Procédé de la revendication 19, dans lequel ledit segment peptidomimétique est un peptoïde.

34. Procédé de la revendication 19, dans lequel ledit segment lieur est choisi dans le groupe consistant en chaînes aliphatiques en C2-C100, poly(oxyde d'éthylène), et oligomères peptidomimétiques ou peptidiques orthogonaux.

35. Procédé de fabrication d'une matrice comprenant une pluralité de différents agents liant des protéines associés de façon stable à la surface d'un support solide, ledit procédé comprenant :
la production d'une banque d'agents liant des protéines, comprenant
un segment d'ancrage de substrat,
un segment peptidomimétique, et
un segment lieur connectant et séparant les segments d'ancrage et peptidomimétique ;
la distribution des agents liant les protéines de ladite banque dans des réceptacles de stockage individuels pour chaque agent liant une protéine différent ;
la préparation d'une pluralité desdits agents liant les protéines pour la liaison sur un substrat solide ;
la préparation d'un substrat solide ayant une surface d'aluminium sensiblement plane pour la liaison avec une pluralité desdits différents agents liant des protéines, l'aluminium étant revêtu d'un revêtement de dioxyde de silicium, lequel revêtement de dioxyde de silicium a une épaisseur d'environ 20 nm à environ 90 nm est capable d'améliorer l'amplification d'un signal fluorescent ;
grâce auquel ladite matrice est produite.

36. Procédé de la revendication 35, dans lequel ladite surface de substrat comprend un revêtement métallique choisi parmi l'or et l'aluminium, et la préparation pour la liaison comprend le nettoyage et le revêtement dudit revêtement métallique avec un aminothiol ou un aminosilane fonctionnalisés.

37. Procédé de la revendication 36, dans lequel ledit aminothiol ou aminosilane est fonctionalisé avec un groupe maléimide.

38. Procédé de la revendication 37, dans lequel ledit segment d'ancrage du substrat est un groupe thiol.

39. Procédé de la revendication 36, dans lequel ladite surface de substrat métallique est en outre revêtue d'une protéine de type avidine en dessous dudit segment d'ancrage.

40. Procédé de la revendication 39, dans lequel ledit segment d'ancrage est la biotine.

41. Procédé de mise en oeuvre d'un test de liaison différentielle, comprenant :
le marquage par fluorescence des protéines dans une solution d'échantillon biologique contenant des protéines ;
la mise en contact d'une fraction aliquote de ladite solution d'échantillon biologique contenant des protéines avec une matrice selon la revendication 1 ;
l'analyse de la matrice afin de déterminer la liaison différentielle des protéines dans l'échantillon aux agents liant les protéines de la matrice.

42. Procédé de la revendication 41, dans lequel ledit segment peptidomimétique est un peptoïde.

43. Procédé de la revendication 42, dans lequel chacun desdits différents agents liant des protéines correspond à un réceptacle source différent contenant cet agent.

44. Procédé de la revendication 43, comprenant en outre la sélection d'un agent liant une protéine d'intérêt à partir des résultats du test de liaison différentielle.

45. Procédé de la revendication 44, comprenant en outre le séquençage du segment peptidomimétique de l'agent liant une protéine sélectionné.

46. Procédé de la revendication 44, comprenant en outre la soumission du segment peptidomimétique de l'agent liant une protéine sélectionné à une analyse structurale par spectroscopie de masse.

47. Procédé de la revendication 44, comprenant en outre l'enrichissement de la solution d'échantillon biologique contenant des protéines avec une protéine qui se lie préférentiellement à l'agent liant une protéine sélectionné, par application d'une seconde fraction aliquote de la solution d'échantillon biologique contenant des protéines sur une colonne de séparation, ladite colonne de séparation comprenant un support de chromatographie présentant l'agent liant une protéine sélectionné.

48. Procédé de la revendication 47, comprenant en outre le séquençage de ladite protéine enrichie.

49. Procédé de la revendication 47, comprenant en outre la soumission de la protéine enrichie à une analyse structurale par spectroscopie de masse.

50. Procédé de la revendication 41, comprenant en outre la mise en contact d'une fraction aliquote de ladite solution d'échantillon biologique contenant un ADNc ou un ARN messager marqué avec une matrice d'ADN, l'analyse de la matrice d'ADN afin de déterminer une liaison différentielle des acides nucléiques dans l'échantillon aux éléments de la matrice d'ADN, et la comparaison des résultats de liaison différentielle des deux matrices afin d'identifier des corrélations entre l'activité des gènes et l'expression des protéine's.

51. Procédé de la revendication 41, dans lequel les marqueurs fluorescents sont des colorants réactifs avec les amines.

52. Procédé de la revendication 51, dans lequel les marqueurs fluorescents sont un ou plusieurs parmi la cyanine 3 et la cyanine 5.

53. Kit destiné à être utilisé dans la mise en oeuvre d'un test de liaison différentielle selon la revendication 41, ledit kit incluant une matrice selon la revendication 1.

54. Kit de la revendication 53, comprenant en outre un ou plusieurs réactifs pour mettre en oeuvre un test de liaison différentielle comprenant une pluralité de marqueurs fluorescents pour protéines.

55. Kit de la revendication 54, dans lequel les marqueurs fluorescents sont des colorants réactifs avec les amines.

56. Kit de la revendication 55, dans lequel les colorants réactif avec les amines sont la cyanine 3 et la cyanine 5.

57. Matrice mixte d'agents liant des protéines attachés de façon stable à la surface d'un support solide, ladite matrice comprenant :
un substrat solide ayant une surface d'aluminium sensiblement plane, revêtu d'un revêtement de dioxyde de silicium, lequel revêtement de dioxyde de silicium a une épaisseur d'environ 20 nm à environ 90 nm et est capable d'améliorer l'amplification d'un signal fluorescent ;
une pluralité de différents agents liant des protéines liés audit substrat, chacun desdits agents liant des protéines comprenant,
un segment d'ancrage lié de façon stable à la surface du substrat,
un segment liant une protéine peptidomimétique, et
un segment lieur connectant et séparant les segments d'ancrage et peptidomimétique ; et
une pluralité de différents anticorps liés audit substrat.

58. Système pour mettre en oeuvre un test de liaison différentielle, ledit système comprenant :
une matrice selon la revendication 1 ;
une ou plusieurs protéines marquées par fluorescence liées à un ou plusieurs des agents liant les protéines ; et
un appareil d'analyse de matrice de signaux fluorescents.

59. Système de la revendication 58, dans lequel les marqueurs fluorescents sont un ou plusieurs parmi la cyanine 3 et la cyanine 5.
